# EUROPEAN PATENT SPECIFICATION

(11) **EP 1 212 107 B1**
(45) Date of publication and mention of the grant of the patent: **08.06.2005**
(21) Application number: 00953780.4
(22) Date of filing: 02.08.2000
(51) Int. Cl.: A61L 27/50, A61L 27/38, A61L 27/24, A61L 27/18, A61L 27/34, A61F 2/06

(54) **ENGINEERED MUSCLE**
REKONSTRUIERTER MUSKEL
MUSCLE ARTIFICIEL

(30) Priority: 31.08.1999 US 386273; 24.02.2000 US 512081
(43) Date of publication of application: 12.06.2002
(73) Proprietor: Virginia Commonwealth University Intellectual Property Foundation, Richmond, VA 23298 (US)
(72) Inventor: BOWLIN, Gary, Mechanicsville, VA 23111 (US); WNEK, Gary, Midlothian, VA 23113 (US); SIMPSON, David, Mechanicsville, VA 23111 (US); TERRACIO, Louis, New York, NY 10012 (US)
(74) Representative: Stevens, Ian Edward
(86) International application number: PCT/US2000/020974
(87) International publication number: WO 2001/015754

(56) References cited:
- EP-A- 0 005 035
- EP-A- 0 266 035
- WO-A-91/01695
- WO-A-97/13849
- GB-A- 2 142 870
- US-A- 4 738 740
- DOSHI J ET AL: "Electrospinning Process and Applications of Electrospun Fibers" JOURNAL OF ELECTROSTATICS,NL,ELSEVIER SCIENCE PUBLISHERS B.V. AMSTERDAM, vol. 35, no. 2, 1 August 1995 (1995-08-01), pages 151-160, XP004040895 ISSN: 0304-3886

## Description

This invention relates to a muscle implant and an extracellular matrix for the implant designed for transplantation. The implant is both a functional and structural replacement for dysfunctional muscle tissue.

### BACKGROUND OF THE INVENTION

Muscle abnormalities are a fact of life whether they result from a developmental anomaly or from a traumatic injury or for any other reason. Structural defects to striated muscle tissue range from relatively functionally benign to profoundly debilitating disorders. In any circumstance, the condition can affect the patient on a number of different levels. For example, structural defects to the musculature of the face may have a minor impact on the ability of a patient to survive. However, even minor cosmetic defects of the muscle of the face can have substantial psychological implications.

In addition to the striated muscle abnormalities noted above, cardiovascular muscle is also subject to deterioration and disease. Congenital malformations of the heart are also common. Conventional surgical techniques are fundamentally unable to adequately restore the subtle structural and functional relationships that exist in a healthy heart. An intact heart has an elaborate three-dimensional structure that insures the orderly propagation of electrical signals and the coordinated contraction of the ventricular wall. If the heart muscle is to be effectively repaired, the three-dimensional organization must be addressed at the cellular level.

Very few alternative technologies exist for the reconstruction of dysfunctional skeletal muscle tissue. Attempts to fabricate such tissue have been generally confined to experiments in which skeletal muscle cells are trapped in a collagen gel. In these experiments, the cells have been seeded onto the exterior of a collagen gel or literally enveloped within the gel as it is polymerized. Subsequently, the cells are allowed to differentiate within the random, "three-dimensional" environment of the collagen gel. The distribution of cells within these collagen gels represents a limiting factor in these constructions. When muscle cells are seeded onto the exterior of a collagen gel they typically remain concentrated on the peripheral regions of the gel. Experiments in which muscle cells are directly incorporated into a collagen gel as it undergoes polymerization have yielded more densely and uniformly populated cultures; however, these constructions remain less dense than their in vivo counterparts. More importantly, the implants produced in conventional tissue culture are composed of muscle cells that lack a uniform alignment or orientation. The random nature of the cells within these sparsely populated implants limits the utility of that tissue and its ability to function as an ordinary muscle.

Additional constraints that must be addressed in designing an implant include the mechanical stability of the implant. The implant must have enough structural integrity to withstand manual manipulation, the surgical procedures and the mechanical environment of the intact tissue. Intact skeletal muscle is surrounded *in vivo* by multiple layers of a dense connective tissue that compartmentalizes the muscle and reinforces the structure of the tissue. Mimicking the specific structure of this arrangement in vitro is difficult, because any dense, investing material will tend to limit nutrient diffusion, oxygen transport and the removal of metabolic waste products away from the cells. Components made from artificial materials such as polyester mesh have been used with some success to increase the strength of the cultures while allowing them to retain a substantial portion of their elastic properties. However, the incorporation of synthetic materials into an implant can increase the likelihood that it will initiate an inflammatory response in vivo.

Cardiac tissue lacks a dense connective tissue. However, the muscle cells of the heart are organized into a complicated lattice. The individual muscle cells of the heart have a rod-like cell shape. Like skeletal muscle, they are oriented along a common axis in a complex three-dimensional pattern. Each cell of the heart is invested with a basement membrane and interconnected to its neighbors by a complex matrix of collagen fibrils. The three dimensional pattern of the cell layers within the heart is critical for the orderly propagation of electrical signals and the coordinate contraction of the ventricular wall.

Smooth muscle surrounds the supports of many of the hollow organs. For example, in the gut it surrounds the stomach and intestinal track. Contraction of this muscle mixes food and propels it along the digestive track. In the cardiovascular system smooth muscle cells surround the walls of the arteries and large veins and functions to control the caliber of the vessels. Smooth muscle lacks the nearly uniform cell shape and lattice like distribution of skeletal and cardiac muscle cells. However, smooth muscle cells do exhibit an elongated, bipolar cell shape. As a population they are organized along a similar axis in a series of overlapping cellular layers. This pattern of organization allows smooth muscle to exert contractile forces in a complex pattern.

### SUMMARY OF THE INVENTION

Accordingly, it is an object of the present invention to overcome the foregoing drawbacks and to provide a muscle implant to a host in need thereof. The implant can be used in a variety of ways including to augment existing muscle, correct muscle deficiencies or as a functional and structural replacement for dysfunctional muscle tissue. Further, the invention includes a method for manufacturing the muscle implant.

In one embodiment, a muscle implant includes an extracellular matrix made of electrospun fibers and muscle cells disposed on the matrix. In another embodiment, the muscle implant comprises an extracellular matrix made of electrospun fibers for supporting muscle, a tendon made of extruded fibers, and a muscle cell layer that is disposed on the extracellular matrix. The muscle cell layer can be multilayered. In other variations, the electrospun fibers may be cross linked. Also, an oriented layer of collagen can be deposited onto the extracellular matrix so that the muscle cells are disposed onto the oriented layer of collagen.

In another embodiment, the invention includes an extracellular matrix for supporting muscle comprising a matrix of electrospun fibers. The fiber is discharged from an electrically charged orifice onto a grounded substrate to form the matrix. The matrix can also be treated with cross linking agents so that the fibers are cross linked.

The invention also includes a method of manufacturing an extracellular matrix comprising extruding electrically charged polymer solution onto a grounded target substrate under conditions effective to deposit polymer fibers on the substrate to form an extracellular matrix. The extruded polymer may form a three-dimensional matrix. The extracellular matrix may further include a gel of aligned collagen fibers deposited thereon.

In a further embodiment, the invention includes a method of forming a muscle fascial sheath by providing an electrically grounded substrate. There is further provided a reservoir of collagen solution wherein the reservoir has an orifice that allows the collagen solution to leave the reservoir. The collagen solution is electrically charged and then streamed onto the substrate to form a muscle fascial sheath.

In still a further embodiment, the invention includes a method of layering muscle cells on an extracellular matrix. The method includes providing an extracellular matrix and then placing the extracellular matrix inside a rotating wall bioreactor. A culture medium is loaded into the bioreactor wherein the medium comprises muscle cells. The bioreactor is then run until muscle cells attach to the extracellular matrix. Alternatively, the muscle cells attached to the extracellular matrix form multiple layers.

An additional embodiment of the invention includes an extracellular matrix comprising a matrix of electroaerosol droplets. The droplets are discharged from an electrically charged orifice onto a grounded substrate to form the matrix. The matrix can also be treated with cross linking agents so that the droplets are cross linked. Additionally, the invention includes a method of manufacturing an extracellular matrix comprising streaming an electrically charged polymer solution onto a grounded target substrate under conditions effective to deposit polymer droplets on the substrate to form an extracellular matrix. The polymer droplets may form a three dimensional matrix. The extracellular matrix may further include a gel of aligned collagen fibers deposited thereon.

Still further, the invention includes a method of forming a vascular prosthesis comprising providing a mandrel about which the prosthesis will be formed. An extracellular matrix is formed by winding a polymer fiber around the mandrel in a helical manner at a predetermined pitch. Muscle cells are then deposited onto the extracellular matrix. This method may further comprise forming a plurality of the extracellular matrix layers around the mandrel. Further, the mandrel may be round or it may be in the shape of a predetermined blood vessel.

### BRIEF DESCRIPTION OF THE DRAWINGS

Figure 1 is a scanning electron micrograph of an electrospun matrix of fibers.
Figures 2A and 2B are schematic drawings of electrospinning devices including the electrospinning equipment and a rotating wall bioreactor.
Figure 3 is a scanning electron micrograph of a thin gel of aligned collagen.
Figure 4A and 4B are scanning electron micrographs of muscle cells deposited on aligned collagen gel and a random collagen gel respectively.
Figures 5A and 5B are schematic representations of how a muscle implant may be fabricated.
Figure 6 is a schematic representation of one type of hypertrophy mechanism.
Figure 7 is a schematic representation of a form into which the polymer stream may be directed.
Figure 8 is a scanning electron micrograph of the external surface of an electroaerosol PGA/PLA (50/50) scaffolding.
Figure 9 is a scanning electron micrograph of a cross sectional view of an electroaerosol PGA/PLA (50/50) scaffolding produced on a 18 gauge needle.
Figure 10 is a scanning electron micrograph cross sectional view of the mid-wall of the electroaerosol PGA/PLA (50/50) scaffolding produced on an 18 gauge needle. This figure illustrates a high magnification of the same construct as shown in Figure 9.
Figure 11 is a scanning electron micrograph of the luminal surface of an electroaerosol PGA/PLA (50/50) scaffolding produced on an 18 gauge needle.
Figure 12 is a schematic representation of five different layers used to create one embodiment of a vascular prosthesis.
Figure 13 illustrates a winding apparatus used to manufacture a vascular prosthesis.
Figure 14 is a schematic of a mandrel with a thin wall tube.
Figure 15 is a schematic of a mandrel holder and mandrel.
Figure 16 is a schematic of a bioreactor composed of tubing connectors, tubing and polycarbonate walls.
Figure 17 is a scanning electron micrograph of a cross-section of a biomimicking vascular prosthetic.
Figure 18 is a scanning electron micrograph showing oriented smooth muscle cells seeded on the external surface of a biomimicking vascular prosthetic.
Figure 19 is a scanning electron micrograph showing oriented smooth muscle cells seeded on the external surface of a biomimicking vascular prosthetic.

### DETAILED DESCRIPTION OF A PREFERRED EMBODIMENT

An engineered muscle implant can comprise one or more of the following components. They are an engineered extracellular matrix, an engineered tendon, and engineered muscle cells. Each component will be discussed in detail separately and then in combination with respect to their assembly.

### A. Engineered extracellular matrix

In a normal human anatomy, muscles are bundles of oriented muscle cells that are encased in an outer protective coating which we refer to as an extracellular matrix. In skeletal muscle, this outer coating is referred to as the fascial sheath. The fascial sheath gives shape and support to the skeletal muscle. The fascial sheath is the extracellular matrix or scaffolding that maintains the integrity of the muscle. Smooth muscle and cardiac muscle are also supported by an extracellular matrix. The cells of smooth and cardiac tissue are both interconnected by a network of collagen fibrils. Smooth muscle lacks a defined fascial sheath. The entire surface of the heart is enclosed in a tough outer coating of connective tissue composed of collagen called the pericardium.

Another type of extracellular matrix is a nerve guide. A nerve guide (or nerve guidance channel) is a tube structure, small bore, which is used to connect the distal and proximal stumps of severed nerve segments to aid in the guidance of the regenerating axons. A nerve guide allows for more direct rewiring of the axons, a direct route for regeneration, and prevents the random axon outgrowths from going off on a tangent and possibly not rewiring. A nerve guide also allows for a controlled environment within the lumen (neuroactive molecules released by the axon stumps) to aid/promote axon regeneration at the same time preventing scar tissue/surrounding tissue ingrowth. The scar tissue/tissue ingrowth would obviously block/prevent axon regeneration.

The engineered extracellular matrix of the present invention can be custom constructed to meet the requirements of skeletal, smooth or cardiac muscles. In preferred embodiments, the extracellular matrix is fabricated by electrospinning polymer fibers or electroaerosoling polymer droplets (synthetic or natural) to form a matrix directly onto a substrate; or to form a matrix directed onto a substrate or form (mold), or other surface such as the central cylinder of a RCCS Bioreactor (Synthecon).

There are a number of different kinds of bioreactors, devices designed to provide a low-shear, high nutrient perfusion environment, available on the market. Until recently, most of the available bioreactors maintained cells in suspension and delivered nutrients and oxygen by sparging, through the use of impellers, or other means of stirring. The RCCS bioreactor is a rotating wall bioreactor. It consists of a small inner cylinder, the substrate for the electrospinning process, positioned inside a larger outer cylinder. Although the electrospun or electroaerosol matrix can be fabricated on the inner cylinder, other locations within the bioreactor also may be used for placement of the matrix for seeding. The gap between the inner and outer cylinders serves as the culture vessel space for cells. Culture medium is oxygenated via an external hydrophobic membrane. The low shear environment of the Synthecon RCCS bioreactor promotes cell-cell and cell-extracellular matrix (ECM) interactions without the damage or "washing away" of nutrients that occurs with active stirring or sparging. Typically, the RCCS device is operated at rotation rates of 8 up to 60 RPM, as required to maintain cells in suspension, and at less than 8 RPM (preferably 2-3 RPM) for cultures immobilized along the center shaft of the vessel. The Synthecon bioreactor can be used in a standard tissue culture incubator.

### 1. Electrospun Extracellular Matrix

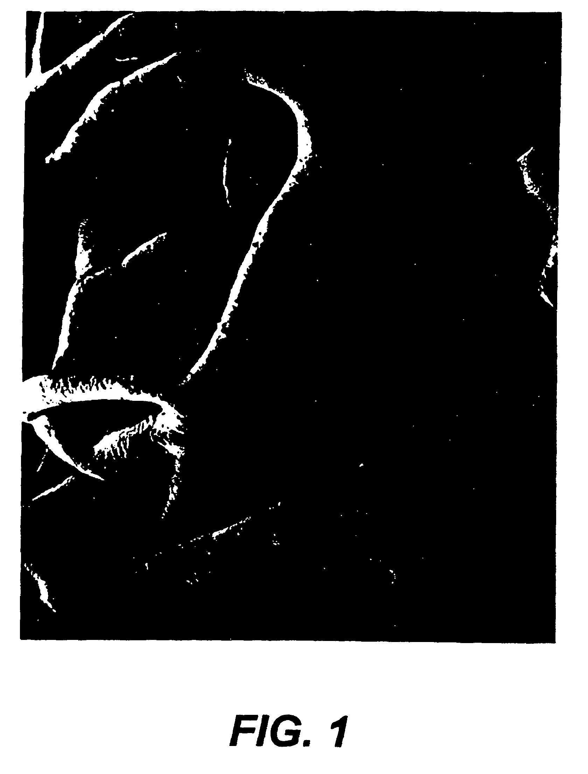

The electrospinning process can be used to produce a dense, mat-like matrix of oriented and/or unoriented polymer fibers (Figure 1). "Electrospinning" means a process in which fibers are formed from a solution or melt by streaming an electrically charged polymer solution or melt through an orifice. Electrospinning has been used in the textile industry to produce ultra thin layers of fiber fabrics (continuous multi filaments) and dense mats of material. The polymer fibers formed by this technique are in the 40-500 nanometer diameter range. The mechanical properties(i.e., strength), porosity, and weight of the fabrics produced by electrospinning can be controlled by regulating the processing conditions, the materials used in the fabrication process and the thickness of the deposited material. Gibson, P.W., et al., Electrospun Fiber Mats: Transport Properties, 1998 AIchE J.; Deshi, J., et al., Electrospinning Process and Applications of Electrospun Fibers, 1996 J. Electrostatics 35:151.

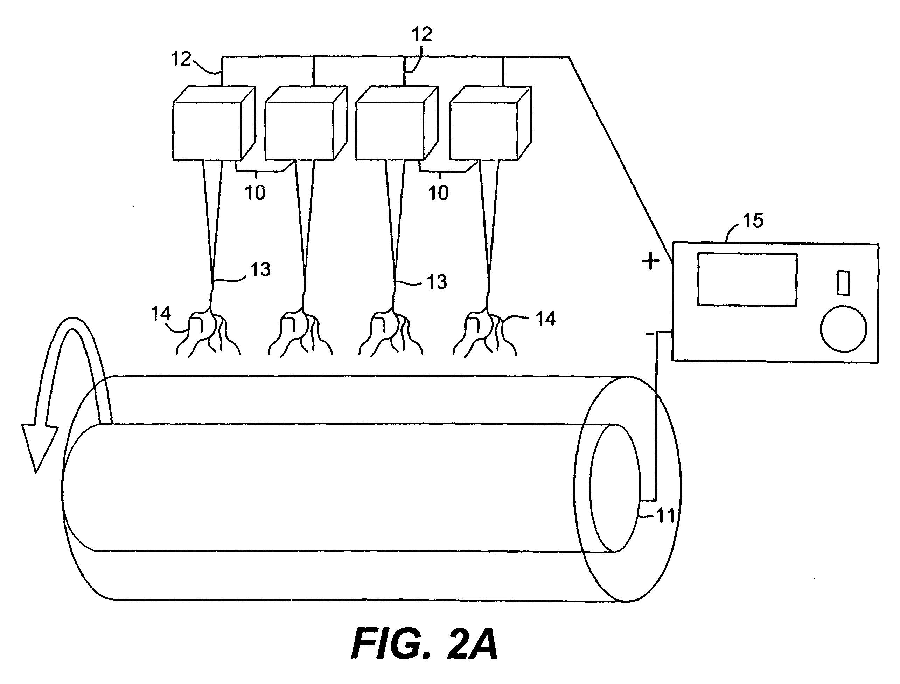

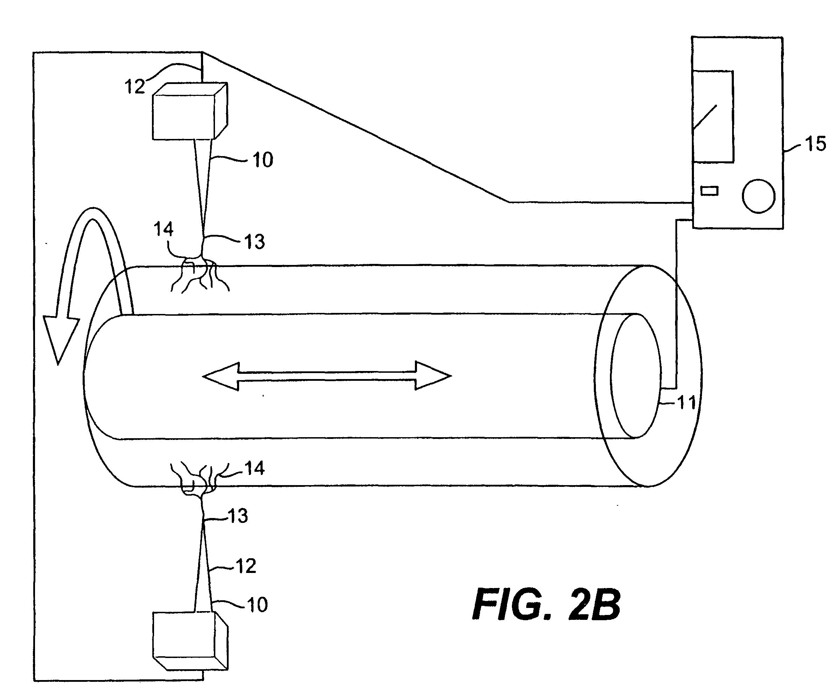

An extracellular matrix of electrospun fibers in accordance with the present invention can be produced analogously. While any polymer can be used, it is preferable to electrospin natural polymer fibers such as collagen fibers. Various effective conditions can be used to electrospin a collagen matrix. While the following is a description of a preferred method, other protocols can be followed to achieve the same result. Referring to Figures 2A and 2B, in electrospinning collagen fibers, micropipettes 10 are filled with a solution of collagen and suspended above a grounded target 11, for instance, a metal ground screen placed inside the central cylinder of the RCCS bioreactor. A fine wire 12 is placed in the solution to charge the collagen solution in each pipette tip 13 to a high voltage. At a specific voltage determined for each solution and apparatus arrangement, the collagen solution suspended in the pipette tip is directed towards the grounded target. This stream 14 of collagen forms a continuous filament that, upon reaching the grounded target, collects and dries to form a three-dimensional, ultra thin, interconnected matrix of collagen (fabric). Minimal electrical current is involved in this process, and, therefore, the streaming process does not denature the collagen, because there is no expected temperature increase in the collagen solution during the procedure.

### 2. Electroaerosol Extracellular Matrix

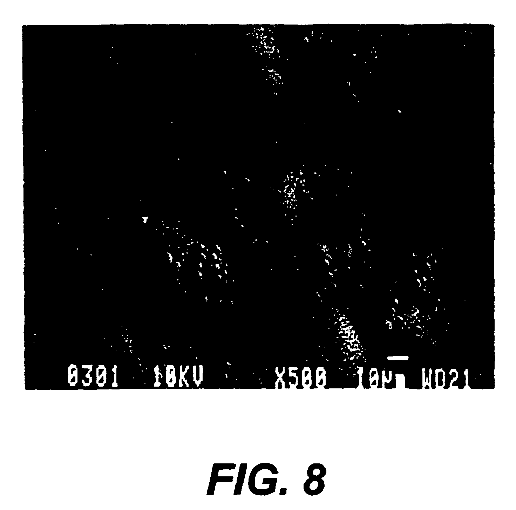

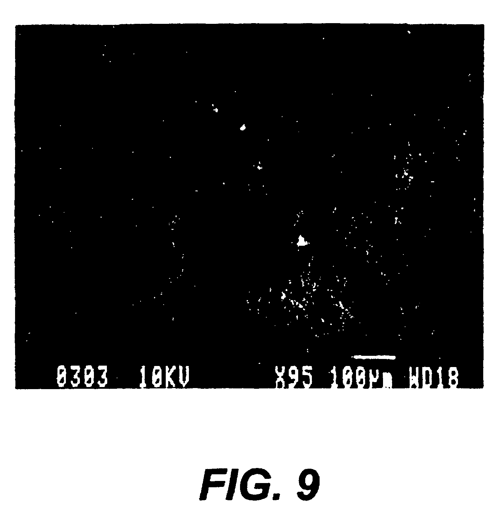

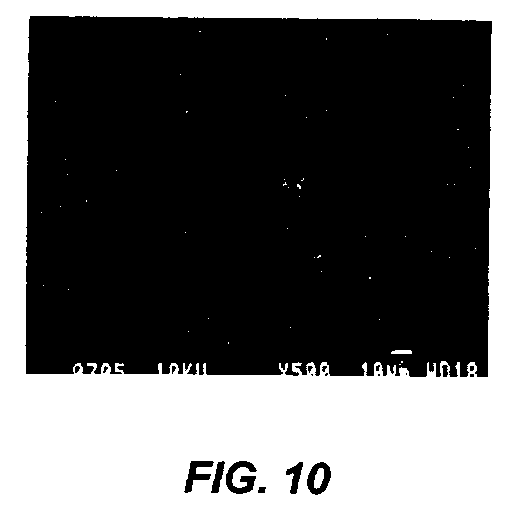

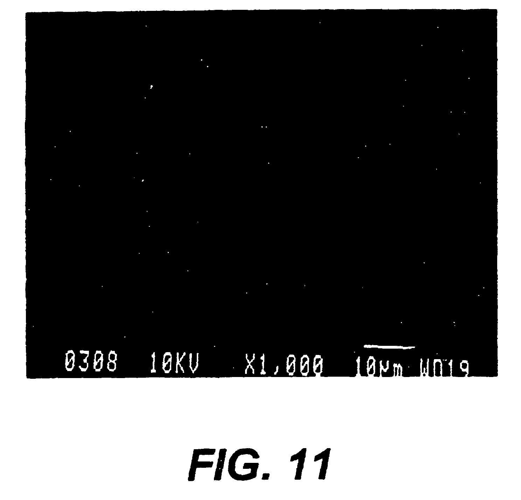

Like the electrospinning process, an electroaerosoling process can be used to produce a dense, matte-like matrix of polymer droplets. "Electroaerosoling" means a process in which droplets are formed from a solution or melt by streaming an electrically charged polymer solution or melt through an orifice. The electroaerosoling process is a modification of the electrospinning process in that the electroaerosol process utilizes a lower concentration of the polymer during the procedure. Instead of producing a polymeric splay (fibers) at the charge tip of the splay nozzle, small droplets are formed. These droplets then travel from the charged tip to the grounded substrate to form a sponge like matrix composed of fused polymeric droplets all on the order of less than 10 microns in diameter. Figures 8 to 11 are scanning electron micrographs of an electroaerosol matrix of 50:50 PGA/PLA extracellular matrix.

As with the electrospinning process described earlier, the electroaerosol process can be carried out using various effective conditions. The same apparatus that is used in the electrospinning process, for instance as shown in Figures 2A and 2B, is utilized in the electroaerosol process. The differences from electrospinning include the concentration of the polymer placed in solution in the micropipette reservoir and/or the voltage used to create the stream of droplets. Obviously, those of skill in the art recognize that changes in the concentration of polymer solution would require modification of the specific voltage to obtain the formation and streaming of droplets from the tip of a pipette.

### 3. Electrospin/Electroaerosol Process Variations

Various polymers can be used alone, or in combination, to produce the electrospun and/or electroaerosol matrices. In preferred embodiments, collagen is used to form the extracellular matrix. Any suitable collagen can be used, including, types I through XIV. In preferred embodiments, types I and III are used. Collagens are available from commercial sources, or they can be prepared according to methods known in the art.

A variety of material can be supplemented into the electrospinning or electroaerosoling solution. DNA coding for desired products (vectors) can be mixed into the polymeric solution for incorporation into the tissue-engineered scaffold. Upon consumption/reorganization of the scaffolding by the seeded cells, they may incorporate the vector (i.e. genetic engineering) into their DNA and produce a desired affect. The DNA can be in any form which is effective to enhance its uptake into cells. For example, it can be naked (e.g., U.S. Patent Nos. 5,580,859; 5,910,488) or complexed or encapsulated (e.g., U.S. Patent Nos. 5,908,777; 5,787,567). Similar to adding DNA, it may be possible to incorporate growth factors or other chemotaxins such as angiogenic factors into the electrospun or electroaerosol matrix to aid in tissue regeneration.

The electrospinning or electroaerosoling process can be manipulated to meet the specific requirements for any given application. The micropipettes can be mounted on a frame that moves in the x, y and z planes with respect to the grounded substrate. The micropipettes may be mounted all around a grounded substrate for instance a tubular mandrel. In this way, the collagen or other polymer streamed from the micropipette can be specifically aimed or patterned. Although the micropipettes can be moved about manually, preferably, the frame onto which the micropipettes are mounted is controlled by a microprocessor and a motor that allows the pattern of streaming collagen to be predetermined by a person making a specific matrix. For instance, collagen fibers or droplets can be oriented in a specific direction, they can be layered, or they can be programmed to be completely random and unoriented.

In the electrospinning process, the polymer stream can branch out to form fibrils of the polymer. The degree of branching can be varied by many factors including, but not limited to, voltage, ground geometry, distance from micropipette tip to the substrate, diameter of micropipette tip, polymer concentration, etc. These variables are well-known to those of skill in the art of electrospinning microfiber textile fabrics.

The geometry of the grounded target can be modified to produce a desired matrix. In a preferred embodiment, a rotating wall bioreactor is used. The grounded target is a cylinder that fits inside the inner cylinder in the electrospinning or electroaerosoling process. By varying the ground geometry, for instance having a planar or linear or multiple points ground, the direction of the streaming collagen can be varied and customized to a particular application. For instance, a grounded target comprising a series of parallel lines can be used to orient electrospun collagen in a specific direction. The grounded target may be a cylindrical mandrel whereby a tubular matrix is formed. Most preferably, the ground is a variable surface that can be controlled by a microprocessor that dictates a specific ground geometry that is programmed into it. Alternatively, for instance, the ground may be mounted on a frame that moves in the x, y, and z planes with respect to a stationary micropipette tip streaming collagen. The grounded target 11 in Figure 2B is shown as being able to oscillate along its longitudinal axis.

The substrate onto which the collagen is streamed can be the grounded target itself or it can be placed between the micropipette tip and the grounded target. The substrate can be specifically shaped, for instance in the shape of a heart or a part thereof or a vascular graft, to substitute or replace a specifically shaped muscle.

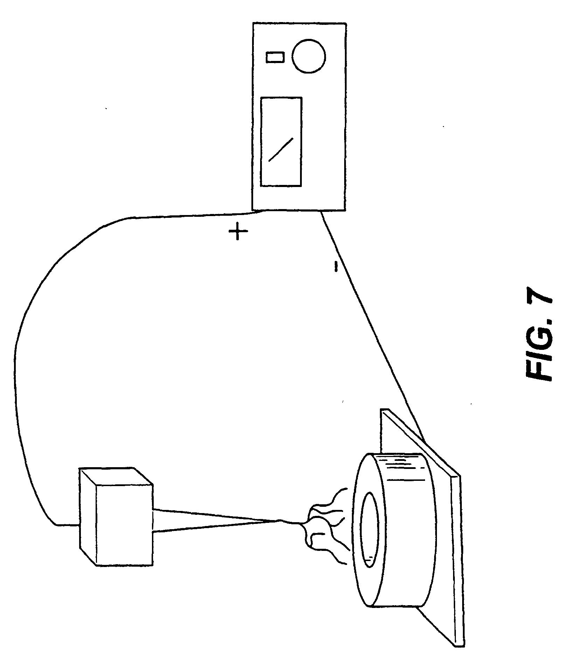

Through modification of a substrate shape and by programming the specific orientation and density of the electrospun or electroaerosol polymer, a very complex muscle pattern can be replicated to enable a specific muscle form for a specific application to be created. Alternatively, for example, collagen may be streamed into a preselected form. The thickness and attributes of the matrix may be preselected to form cartilage, dentin packing, or other similar prosthesis. A schematic example of this type of application is shown in Figure 7.

Other variations on electrospinning and electroaerosoling include:
1. Using different solutions (e.g.,. collagen I and III) to produce two or more different fibers or droplets simultaneously (matrix fiber or droplet array). In this case, the single component solutions can be maintained in separate reservoirs.
2. Using mixed solutions (e.g., collagen I and III) in the same reservoir(s) to produce fibers or droplets composed of multiple polymers (fiber composition "blends"). Nonbiological but biologically compatible material can be mixed with a biological molecule such as collagen, e.g., PVA, PLA, PGA, PEO, etc.
3. Utilizing multiple potentials applied for the different solutions or even the same solutions.
4. Having two or more different geometric grounded targets (i.e. small and large mesh screens).

All these variations can be done separately or in combination with each other to produce a wide variety of electrospun and electroaerosol extracellular matrices.

Additionally, a matrix can be formed that includes both electrospun and electroaerosol polymers. In other words, a combination of fibers and droplets may be beneficial for some applications envisioned by a particular structure to be mimicked. This combination of fibers and droplets can be obtained by using the same micropipette and solution in varying the electrical charge; varying the distance from the grounded substrate; varying the polymer concentration in the reservoir; using multiple micropipettes -- some from streaming fibers and others for streaming droplets; or any other variations to the method that could be envisioned by those of skill in this art. The fibers and droplets could be layered or mixed together in same layers. The possible combinations of the electrospinning and electroaerosoling processes are virtually unlimited.

The stability, rigidity, and other attributes of the electrospun or electroaerosol matrix can be regulated by the degree to which it is chemically modified. The electrospun or electroaerosol matrix may be used in its unmodified state, or it may be modified in accordance with the requirements of a specific application. Modifications to the matrix can be made during the electrospinning or electroaerosoling process or after it is deposited. Cross-linking agents such as carbodiimide EDC (1-ethyl-3(3 dimethyl aminopropyl)), carbodiimide hydrochloride, NHS (n-hydroxysuccinimide), or UV light can be used, e.g., to stabilize the fascial sheath against proteolytic attack, and/or to increase the stability of collagen gels. See, e.g., Van Wachem, et al., 1996 Myoblast seeding in a collagen matrix evaluated in vitro, J. Biomedical Materials Res. 30:353-60.

### 4. Biomimicking a Vascular Prosthesis

One type of engineered extracellular matrix and/or engineered muscle (media component) that can be fabricated is a vascular prosthesis. A successful prosthesis may be accomplished best if the prosthesis biomimics the artery or other vessel or branch that is to be replaced. One of the keys to creating this prosthesis is the orientation and layering of the extracellular matrix that will be the scaffolding for the prosthesis.

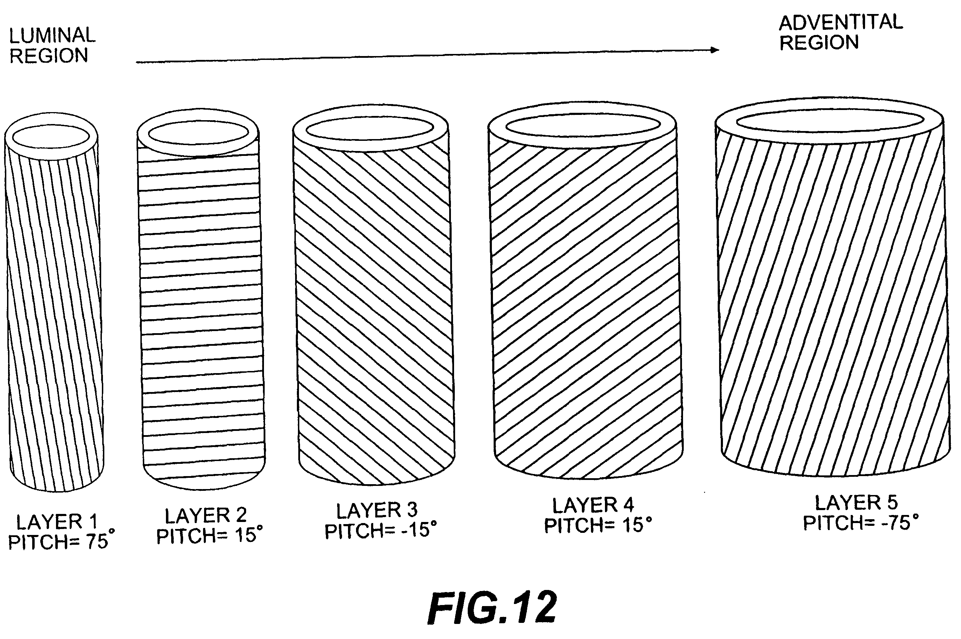

The vascular prosthesis includes winding thin collagen fibers (<150 µm) in a collagen gel and/or in a matrix of electrospun or electroaerosol polymer(s). Although any polymer may be used, natural polymers are preferable. In a specific example using electrospun PLA fibers, a matrix of the electrospun fibers are formed around a thin mandrel. The collagen fibers are then wound in a helical manner around (embedded in) the matrix of electrospun fibers. The composite of collagen fiber and electrospun matrix creates a residual stress environment similar to that found in native arteries. Multiple layers of the wound fibers and electrospun matrices can be assembled with each layer of the multi-layer prosthesis oriented at a specific pitch to mimic the specific vasculature being replaced. The specific application will also determine the number and thickness of the layers. In one embodiment addressed to building a small diameter artery, each layer is cylindrical with each possessing a collagen fibril bundle/smooth muscle cell structure wound (spiral arrangement) at varying pitches (Figure 12 displays a 5 layer prostheses). All historical attempts at developing a vascular construct from collagen and cellular components have been unsuccessful for two reasons: 1) the collagen was added in the form of a gel containing smooth muscle cells and allowed to form around a solid mandrel (no specific orientation) and 2) the procedures were maintained at static conditions.

The polymer (preferably collagen and/or other natural polymers) that makes up each layer can be oriented using the electrospinning and/or electroaerosoling techniques described herein. Alternatively, as described in Example 4, the layers may be formed on a mandrel by mechanical winding of fibers about the mandrel at specified pitches. Still further alternatively, a combination of mechanical winding and electrospinning/electroaerosoling may be used to assemble the extracellular matrix.

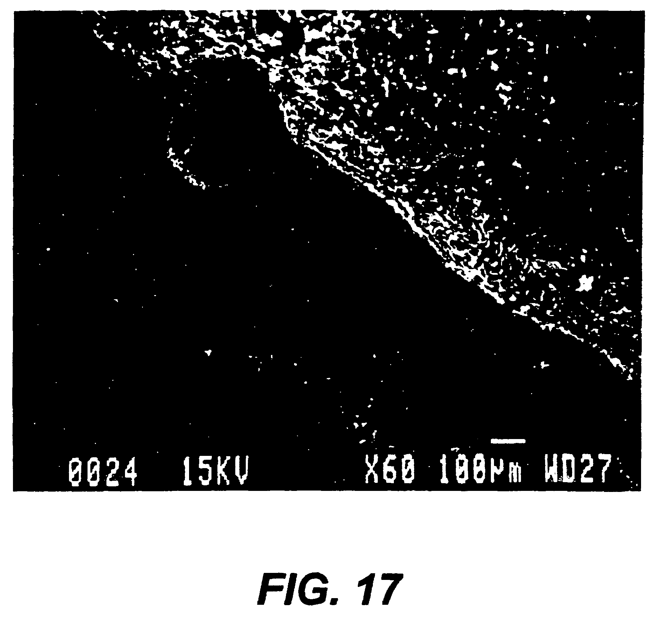

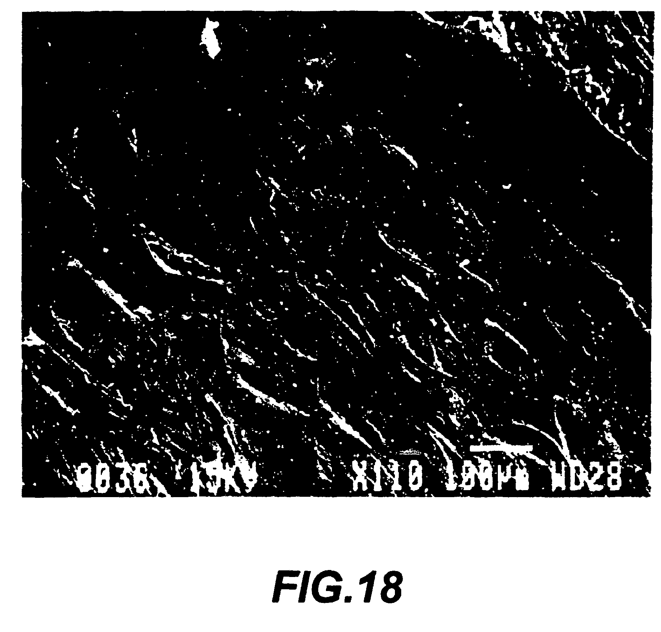

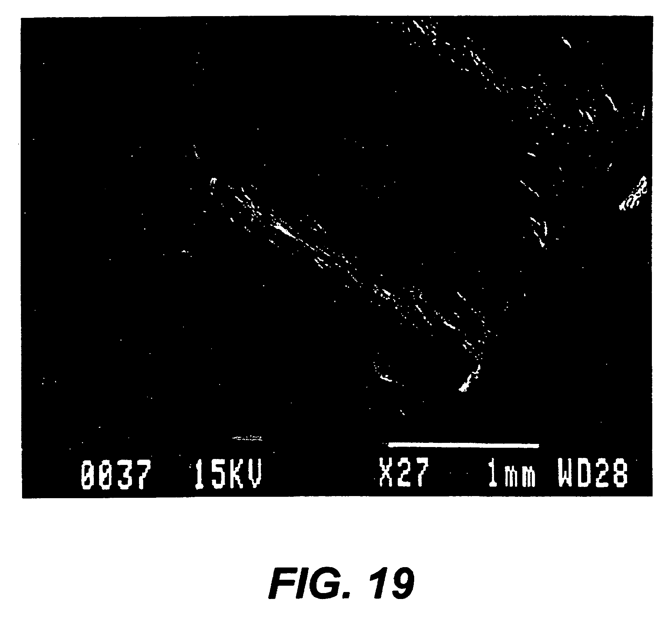

It has been discovered that the mechanical winding of polymer fibers in the prosthesis has an unexpected effect on muscle cells subsequently seeded on the extracellular matrix. The residual stress in the wound polymer (collagen) creates an energy in the prosthesis that promotes growth of the muscle cells in the direction of the spiral wound polymer. This residual stress also mimics the actual stress found in natural arteries. Figures 17-19 display a matrix and wound fiber composite as well as the orientation of muscle cells seeded onto the prosthesis.

Also, the specific embodiment described in Example 4 is directed to a simple, tubular vascular graft. More complicated shapes including tapered and/or branched vessels may also be constructed. All that is necessary is a different-shaped mandrel to wind the large fibers around or to orient the electrospun/electroaerosol polymer around.

### B. Engineered tendon.

The engineered tendon, or the connective tissue struts that anchor the engineered muscle to bone, can be assembled from extruded collagen fibers or other suitable materials. Collagen fibers are preferred, because collagen is less likely to be rejected by a recipient's immune system. These fibers function in combination with the extracelluar matrix to stabilize the overall structural integrity of the muscle implants. Collagen fibers for the fabrication of the engineered tendon can be extruded after known methods. Kato, Y.P. and Silver, F.H., Formation of Continuous Collagen Fibers: Evaluation of Biocompatibility and Mechanical Properties, 1990 Biomaterials 11:169-75; Kato, Y.P., et al., Mechanical Properties of Collagen Fibers: A Comparison of Reconstituted Rat Tendon Fibers, 1989 Biomaterials 10:38-42; and U.S. Patent Nos. 5,378,469 and 5,256,418 to Kemp, et al..

A preferred collagen extrusion apparatus comprises a syringe pump, microbore tubing, a dehydration trough, recirculation pump, rinsing trough, drying chamber, heating air dryer, and a collagen fiber winder. The syringe is filled with degassed collagen and mounted onto a syringe pump. The collagen solution is then extruded from the syringe, through the microbore tubing, and into a dehydration bath (Polyethylene glycol in PBS). The formed collagen fiber is subsequently guided through a rinsing bath (phosphate buffered saline, PBS) and attached to a winding system within a dryer. Once the initial fiber has been formed and attached to the winding element, the process becomes automated and continuous. At an extrusion rate of approximately 8 cm/minute, the extrusion apparatus can produce fiber 1-10 meters in length and 50-250 µm in diameter. After production, the fiber diameter can be verified through scanning electron and light microscopic evaluation. Varying the reaction conditions controls the diameter of the collagen fiber that is polymerized. The physical properties of the engineered collagen fiber can be further modified and controlled by regulating the composition of the extrusion material. The elastic properties of the engineered tendon can be modulated by incorporated elastin, fibrin or man made material into the collagen solution as it is extruded. Prior to use in the engineered implant the collagen fibers are sterilized by peracetic acid sterilization.

### C. Engineered muscle cells

Any type of muscle cells can be used in the present invention, including cell culture strains, transformed cells, primary muscle cells, non-human embryonic muscle cells, neonatal muscle cells, non-human embryonic stem cells, etc. Preferred cells are stem cells or muscle cells (or muscle precursor cells) which are obtained from a host into which the muscle will be transplanted. Barrofio, A., et al., Identification of Self-Renewing Myoblasts in the Progeny of Single Human Muscle Satellite Cells, 1996 Differentiation 66:47-57; Blau, H.M. and Webster, C., Isolation and Characterization of Human Muscle Cells, 1981 Proc. Nati. Acad. Sci 78:5623-27. The term "primary myocytes" means muscle cells which are obtained directly from a host animal muscle which retain the ability to differentiate and which have been passed a minimum number of times in culture. Such cells generally are not transformed.

This application does not include the use of human embryonic material.

The cell type to be used in the implant depends upon use and the site of implantation that is to be reconstructed repaired or otherwise augmented by the engineered muscle. A variety of cell types can be used and include but are not limited to; non-human embryonic stem cells, bone marrow stem cells, satellite muscle cells from the striated muscle beds, cardiac muscle cells, smooth muscle cells, muscle cell lines, transformed cell lines and genetically engineered cell lines. Cells isolated from fetal, neonatal and adult tissue may be used. Fetal cardiac myocytes can be used in the construction of the cardiac prosthesis. Cells of the C2C12 muscle cell line can be used for the fabrication of skeletal muscle implants. In the long run a stem cell population (adult or non-human embryonic) will be the ideal cell source for the fabrication of the muscle. Stem cells are attractive for this use because they can be engineered to become nearly any type of cell (e.g., smooth muscle, cardiac muscle, skeletal muscle, cartilage, bone, etc). They can but do not have to come from the patient to be treated with the muscle implant, because even if they come from some other source they will not invoke an immune response.

For skeletal muscle, satellite muscle cells are derived from a suitable, and unobtrusive, donor site on the subject who is to receive the muscle implant. Muscle biopsies are isolated, and the connective tissue removed by dissection. Various protocols can be utilized to isolate satellite or other cell types from the muscle for engineering the implant. For instance, a protocol can be as follows: Isolated muscle tissue is minced and dissociated into a single cell suspension, by trypsin-EDTA digestion (or other suitable enzymes) under constant stirring. At the conclusion of the enzymatic dissociation procedure, the digestion media is quenched with the addition of 10% serum. Cell suspensions are washed by centrifugation, and a sample is stored in liquid nitrogen for future use. The balance of the isolated cells are enriched in satellite cells by flow cytometery or through the use of differential adhesion or through immunoseparation methods. Flow cytometery, can be used to separate cells by size, by cell cycle stage, or, if labeled, by cell surface markers. In differential adhesion, cells are placed into a culture vessel for a short incubation period to allow contaminating fibroblasts to seed out of the solution, thus enriching the remaining cell population in satellite cells. A longer interval of differential adhesion can be used to seed out satellite cells so they can be purified away from contaminating debris and muscle cells. Enriched cell fractions that contain myogenic satellite cells can be stored at -70°C in liquid nitrogen. Regardless of the technique used to isolate and purify satellite cells the samples are thawed and plated into culture vessels and assayed for myogenic potential. Cell lots that are competent to undergo differentiation are used in the fabrication of the muscle implant. After partial purification, clones are assayed for myogenic potential, e.g., by plating cells onto collagen - coated dishes and observing whether adherent cells display the characteristics of muscle cells.

Candidate clones from the primary cell isolate are grown under sparse culture condition (i.e. low cell density) in an appropriate media, e.g., containing 10-15 % serum, to accumulate an adequate number of cells from which the implant can be fashioned. Once a sufficient number of cells have been obtained (dependent upon the size of the implant to be fabricated), they are prepared for insertion into the bioreactor for the assembly of the prosthetic muscle. Muscle cell differentiation in the bioreactor can be induced by replacing the high serum content media (10-15% serum) with low serum media.

Cells utilized in the fabricated muscle are readily amenable to genetic manipulation. For example, genes encoding angiogentic factors, growth factors or structural proteins can be incorporated into the isolated cells. This can be accomplished before, during or after the fabrication of the muscle construct. Useful genes include, e.g., VEGF, FGF, and related genes. For introducing genes into cells, any effective method can be used, including viral vectors, such as adenovirus, also DNA, plasmids, etc. can be used.

### D. Assembly of an engineered skeletal muscle implant

The muscle implant can comprise three distinct components, the extracellular matrix, the engineered tendon and the population of muscle cells. As detailed earlier, an extracellular matrix composed of a matrix of collagen fibers or other biologically compatible material is prepared on the outer surface of the inner cylinder of an RCCS bioreactor (or suitable substitute). The structural properties of this mat of fibers are regulated by the diameter of fibers produced, the relative concentration of materials used in the reaction (e.g. concentration of type I to type III collagen, or other incorporated materials), and other reaction conditions.

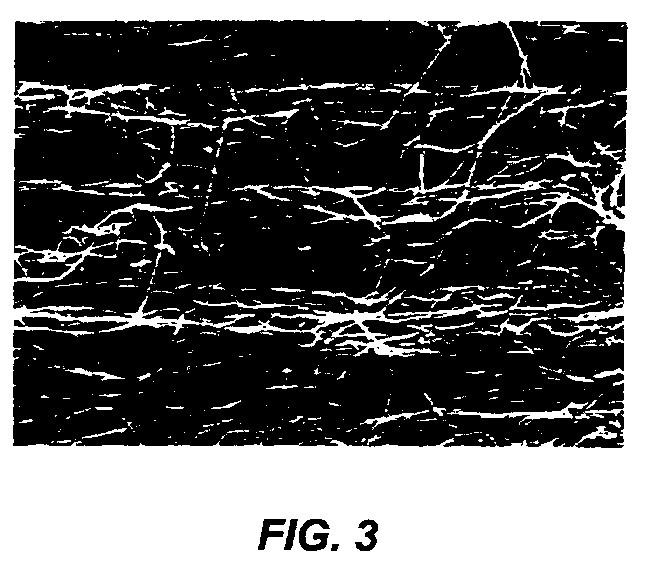

In one preferred embodiment, a thin gel matrix of collagen or other suitable matrix material can be applied over the surface of the extracellular matrix to enhance muscle cell adhesion, differentiation, and/or alignment. The gel matrix can be applied in any suitable manner including electrospinning, spraying, dipping, spreading, dropping, etc. Simpson, et al., Modulation of Cardiac Phenotype in vitro by the Composition and Organization of the Extracellular matrix, 1994 J. Cell Physiol. 161:89-105. In a preferred embodiment, the collagen fibers in the thin gel are aligned along a common axis. For example, the aligned matrix can be produced by dipping the central cylinder core of the RCCS bioreactor, with its electrospun coating of collagen, end-on into a ice cold neutral stock solution of collagen (1 mg/ml) (Type I or type III or a mixture thereof). After a very brief interval (1-3 secs), the cylinder is removed from the solution and the excess collagen is allowed to drain by gravity off of the distal end of the cylinder. The orientation of the cylinder is maintained constant throughout this process, i.e. perpendicular to the collagen solution in which it was dipped. This allows the excess collagen to drain off the long axis of the cylinder. The cylinder is then placed into an incubator, e.g., set for 37°C, to allow the collagen to polymerize, e.g., sixty minutes or more. After polymerization is complete, the aligned collagen fibers are allowed to dry down on to the underlying facial sheath. These procedures result in a thin layer of aligned collagen fibrils arrayed along the axis the cylinder was drained. See Figure 3. Other methods for aligning the collagen may be employed, for instance, using the described electrospinning system or using a centrifuge after dipping the core in the collagen solution. Regardless of how the collagen is aligned, at the conclusion of this step, the central RCCS cylinder has a mat-like coating of electrospun collagen fibers (the extracellular matrix) covered or coated with a thin layer of aligned collagen.

If desired, the extracellular matrix can incorporate other materials as well, such as polyester mesh and other synthetic materials.

Also, as discussed earlier, the need for the thin gel of collagen fibers may be obviated if the electrospun matrix is sufficiently oriented during the electrospinning process. In other words, the additional thin gel layer of oriented collagen is only necessary if the extracellular matrix (fascial sheath in the example of skeletal muscle) of collagen or other polymer is unoriented.

Large diameter, extruded collagen fibers (engineered tendons) are then applied over the aligned collagen gel. The mechanical properties of the implant are controlled in this step at two separate sites. First, by the thickness of the individual extruded fibers and the number of these filaments added to the implant. Second, by the orientation of these fibers with respect to the long axis of prosthesis. The implant can be made more or less stiff by applying these fibers in an undulating pattern. The large fibers can also be attached to the matrix by only overlapping the matrix at the distal ends, i.e., not necessarily running the entire length of the engineered muscle. Regardless of the orientation used, the ends of extruded fibers are allowed to project from the distal ends of the implant. At the conclusion of this step, the large diameter collagen fibers are allowed to dry down onto the fibers of the aligned collagen gel. Alternative fabrication processes can be used to further customize the mechanical properties of the implant. For example, large diameter collagen fibers may be laid down first followed by collagen fibers deposited by electrospinning, followed by another layer of large diameter collagen fibers, the aligned collagen gel and the satellite cells. Other permutations on this assembly process are also possible.

A tendon can also be created in situ by combining tendon fibroblasts with the synthetic muscle bed. For example, tendon fibroblasts may also be harvested from a recipient's own tendon. These cells are placed on the end of the muscle bed synthesized as described herein. The tendon is allowed to grow with the muscle bed in the bioreactor. The tendon fibroblasts are encouraged to grow in an oriented fashion by use of the aligned substrate herein or by other orientation methods. If this method is chosen, the extruded collagen tendons described herein become unnecessary, although a combination of extruded and cultured tendons may be desired for certain applications.

In the final step of the fabrication process, the inner cylinder with its engineered fascial sheath and overlaying layers of aligned collagen and large diameter collagen fibers is loaded into a RCCS bioreactor. Muscle cells, such as satellite myoblasts isolated from the subject or compatible donor, are loaded into the chamber and allowed to interact with the collagen-based substrate. The RCCS bioreactor is preferably used in this step because it provides high nutrient profusion in a very low shear environment. However, other culture vessels can be used. Under these conditions, it is possible to assemble a muscle cell culture comprising multiple layers (8-12 layers in 48 hours) of aligned cells. In the assembly of the muscle implant, cells are gradually depleted from suspension culture and plated onto the collagen matrix, either directly on the electrospun matrix or on the collagen gel coating, to form the three dimensional arrangement of the engineered tissue. Additional satellite cells are added as need to the bioreactor to assemble additional cell layers. Once the desired mass of cells has been plated onto the fascial sheath, they are allowed to differentiate into myotubes, e.g., by transfer to a serum media. Also, there are artificial oxygen carriers that can be used in vitro to increase oxygen delivery to tissues or cells in culture. They would be mixed into the reactor with the satellite cells as the muscle is fabricated. They basically function like red blood cells in the sense these carriers increase the oxygen content of the culture media.

Any suitable culture media can be used to grow the cells, including medias comprising serum and other undefined constituents, defined medias, or combinations thereof, RPMI, etc.

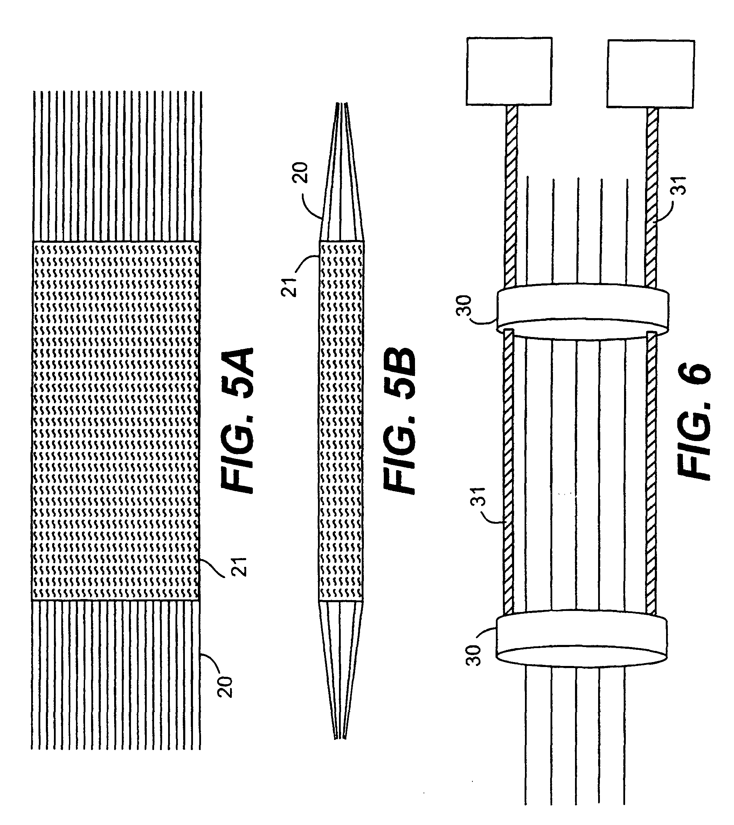

With the completion of the differentiation process, the skeletal muscle implant is ready for transplantation into the site of reconstruction in the subject. The implant is removed from the central cylinder of the bioreactor. The size and thickness of the implant is controlled at this stage of the process at two different levels -- first, by the number of cell layers assembled onto the central cylinder and, second, by the size of the sheet of tissue used to construct the muscle implant. This latter procedure involves trimming (for instance, into a rectangular sheet - Figure 5A), stacking, and rolling (Figure 5B) the engineered muscle into the desired configuration. Alternatively, the engineered muscle can be directly implanted or layered for the reconstruction of, for instance, facial muscles as a flat sheet. If the engineered muscle is to be used to reconstruct a muscle bed of the axial skeleton, it may be attached to the implantation site through the large diameter collagen fibers 20 that protrude from the ends of engineered muscle 21, through the distal ends of the fascial sheath itself or through a combination of these methods.

If the tissue is to be used to reconstruct a congenital heart defect or repair an otherwise dysfunctional region of myocardium or reconstruct a muscle of facial expression it can be sutured or affixed in place with fibrin glue. By modifying the assembly process, implants for the reconstruction of cardiac muscle or smooth muscle can be assembled. In general, the major modification may be in the relative pattern of the engineered extracellular matrix and connective struts or tendons described in this application. Cardiac tissue and smooth muscle lack tendons. However, the use of large diameter collagen fiber may still be desirable to lend mechanical strength to the implant. In the case of cardiac implants, the large fibers may be used as a delivery system to assemble or to manipulate the implants. The key common feature to assembly of these implants is the ability to fabricate a multi-layer implant composed of cells in an in vivo pattern of organization.

Vascularization of the implanted muscle tissue will occur in situ several days after surgery. It can be stimulated further, as mentioned above, by angiogenetic and growth-promoting factors, either administered as peptides or as gene therapy. Another alternative for supplying engineered tissue with a vascular supply is to temporarily transplant the muscle into the omentum. The omentum has an extensive and rich vascular supply that could be tapped and used like a living incubator for the support of engineered tissue. The engineered tissue would be removed from a bioreactor and wrapped in the omentum and would be supported by the diffusion of nutrients and oxygen from the surrounding tissue. Alternatively or in addition to this approach, engineered tissue could be connected directly to the endogenous vascular supply of the omentum. A blood vessel might be partially perforated or cut or left simply dissected free of the omentum. The engineered tissue could then be wrapped around the vessel. The engineered tissue would be supported by nutrients leaking from the perforated vessel or by the simple diffusion of nutrients if the vessel was left intact. Regardless of strategy, the engineered tissue would be surrounded by the omentum and its rich vascular supply. It is also possible to engineer muscle with an endogenous vascular system. This vascular system might be composed of artificial vessels or blood vessels excised from a donor site on the transplant recipient. The engineered tissue would then be assembled around the vessel. By enveloping such a vessel with the muscle during or after assembly of the engineered muscle, the engineered tissue would have a vessel that could be attached to the vascular system of the recipient. In this example, a vessel in the omentum would be cut. The vessel of the engineered muscle would be inserted and connected to the two free ends of the omental vessel. Blood would pass from the omental vessel into the vascular system of the muscle, then pass through the muscle and drain back into the omentum vessel. By wrapping the tissue in the omentum and connecting it to a omental blood vessel, the engineered tissue would be supported by the diffusion of nutrients from the omentum and the vessel incorporated into the muscle during its fabrication. After a suitable period of time the muscle would be removed from the omental "incubator" and placed in the correct site in the recipient. By using this type of strategy the engineered muscle could be supported in a nutrient rich environment during the first several days following its removal from the bioreactor. The environment of the omentum also has the capacity to promote the formation of new blood vessels in implanted tissue. This omental incubator strategy could also be combined with the other angiogenic strategies discussed earlier.

The engineered muscle described above is advantageous in several respects. First, the connective backbone support of the implant comprises natural materials. This material has low antigenic potential and its structural properties can be regulated at many different sites, including, but not limited to; the relative concentration of different collagen isoforms used to produce the sheath, the thickness of the fibers used and, the degree of chemical cross-linking present in the matrix. Next, the implant uses large diameter collagen fibers to further modify the structural properties of the implant and provide a means to anchor the engineered muscle to the site of transplantation. These fibers are very similar to the fibers used to manufacture catgut for surgical sutures (>250 µm), however, the extrusion process allows for better control of fiber diameter and the fabrication of fibers that are much smaller in diameter than conventional catgut (50 to 200 µm depending upon reaction condition). Preliminary studies from other laboratories indicate the efficacy of using extruded collagen fibers in the production of tendons in the rat and in the formation of woven sheets for the repairs of experimental abdominal wounds in the rat. The implantation of large diameter, extruded collagen fibers did not induce inflammation beyond background levels in these experiments.

The stem cells or muscle cells used to construct the implant can be isolated from the subject, or other compatible donor, that requires muscle reconstruction. This has the obvious advantage of using cells that will not induce an immune response, because they originated with the subject (autologous tissue) requiring the reconstruction. Relatively small muscle biopsies can be used to obtain a sufficient number of cells to construct the implant. This minimizes functional deficits and damage to endogenous muscle tissues that serves as the donor site for satellite muscle cells.

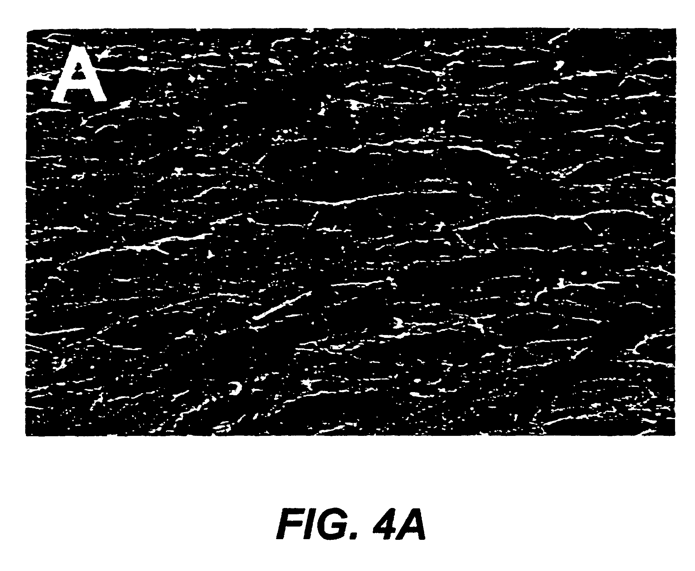

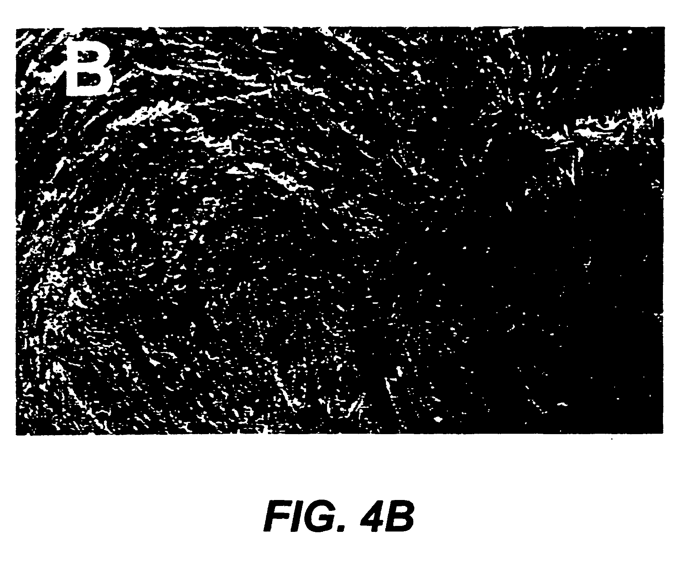

Another factor unique to this muscle prosthesis is the shape of the individual muscle cells and their three-dimensional arrangement. The cultures are composed of linear, three-dimensional arrays of muscle cells distributed along a common axis in an in vivo-like pattern of organization. This makes it possible to install an implant that can produce contractile force along a defined direction, allowing for the structural and functional repair of a dysfunctional muscle bed. This feature is especially critical in cardiac muscle where it is essential to restore both the mechanical and electrical properties of the damaged muscle. Cultures prepared on, or, in a random gel of collagen lack this uniform alignment. Compare Figures 4A (aligned) with Figure 4B (random). Random cultures are unsuitable for the use in reconstruction because they lack the clearly defined orientation that is characteristic of intact skeletal muscle and the individual cellular layers of the heart. The highly polarized nature of intact muscle allows it to effectively and efficiently apply mechanical force along a defined axis during contraction. This property also facilitates the conduction of electrical impulses through the tissue.

Strategies must be implemented to promote the formation of vascular elements within the implant. Several options are available. First, the implants can be seeded with angioblasts and/or endothelial cells to accelerate the formation of vascular elements once the engineered tissue is placed in situ. Second, angiogenic peptides can be introduced into the engineered tissue via an osmotic pump. The use of an osmotic pump makes it possible to deliver active peptides or, as noted, angiogenic peptides or growth factors directly to the site of interest in a biologically efficient and cost-effective manner. Experimentation in the vascular bed of ischemic skeletal muscles has demonstrated the efficacy of this approach (Hopkins et al., Controlled delivery of vascular endothelial growth factor promotes neovascularization and maintains the limb function in a rabbit model of ishemia, 1997 J. of Vascular Surgery 27:886-95). VEGF delivered to ischemic hind limbs of rabbits accelerated capillary bed growth, increased vascular branching and improved muscular performance with respect to ischemic controls. Upon initial implantation, an early phase of muscle degeneration of intact muscle implants (Faulkner et al., Revascularization of skeletal muscle transplanted into the hamster cheek pouch: Interavital and light microscopy, 1983 Microvasular Res. 26:49-64) suggests that it may be desirable to implant engineered muscle tissue at a time just prior to muscle differentiation. An alternative approach is to "seed" fully differentiated muscle constructs with additional satellite cells and/or endothelial cells and or angioblasts shortly before they are implanted in situ. Also, use of the omentum "incubator" was discussed earlier.

In vivo deneravation of skeletal muscle promotes the evolution of atrophy in the effected tissue. To a great extent, this response appears to develop because deneravation reduces the amount of resting tension observed in the affected muscle (Thomsen and Luco 1944; Gutman et al., 1971). In vitro, the effects of denervation may be substantially overcome by applying tension to the denervated muscle. Cardiac muscle is also very sensitive to its surrounding mechanical environment. Muscle mass may be initially retained in an engineered prosthesis by placing it under tension at the time it is implanted. Different methods of promoting hypertrophy or stretching of the implant are available. A mechanical stretcher can be used in the bioreactor by attachment of the "tendons" on either end of the implant to ring clamps to tighten or loosen the implant. Figure 6 illustrates how the fibers will be held in place (at desired orientations longitudinally) on the inner cylinder surface of the bioreactor by two end supports 30. Figures 6 also illustrates the use of motor driven screw 31 drives to be added to the bioreactor to allow mechanical stretching (well defined percentage of stretch) for preconditioning particular tissue (muscle, blood vessels, and intestines) during the initial cell seeding/development stage. The stretching makes bigger, thicker and stronger cells/tissue that are less likely to tear after implantation. The stretching can also be used to further align the muscle cells. Electrical pacing or pharmacological stimulation can also be used. Electrical pacing, in particular, is very effective and easy to control.

The second level of control that is imparted by the central nervous system on skeletal muscle is more fundamental. Neural inputs directly control the action of the tissue. In order to achieve a fully functional muscle prosthesis it is necessary to bring it under the control of the central nervous system. Preferably, the engineered implant can be transplanted into a muscle bed adjacent to the area of interest and allowed to adapt to the in vivo environment. After a period of adaptation the autologous implant would be mobilized, perhaps with a portion of the motor units arising from the original transplant site, and repositioned within the site requiring reconstruction. It may also be possible to induce the ingrowth of motor neurons through the use of growth peptides delivered by osmotic pumps, or other means, to the implant tissue. Cardiac tissue mass is not subject to much regulation by the central nervous system. However, it is sensitive to changes in mechanical activity. By pre-stressing by stretching, electrical stimulation or using pharmacological agents to promote cardiac muscle hypertrophy an implant of cardiac muscle during or following the fabrication process, it can be better prepared for the region of the in vivo environment.

### Example 1 (Electrospinning an Extracellular Matrix)

An extracellular matrix was made of poly-lactic/poly-glycolyic acid (PLA/PGA; 50/50 - RESOMER® RG 503, Boehringer Ingelheim, Germany) and poly(ethylene-co-vinyl) acetate (Aldrich Chemical Company, Inc., Milwaukee, WI) polymers. The concentration of the two polymers dissolved in dichloromethane (Sigma-Aldrich, St. Louis, MO) were 0.19 g/ml RESOMER® RG 503 and 0.077 g/ml poly(ethylene-co-vinyl) acetate. The electrospinning set-up consists of a glass pipet (overall length approximately 21 cm with a tapered tip with an opening estimated at 0.3 mm, no exact measurement obtained, 0.32 mm diameter silver-coated copper wire, 20x20 mesh 316 stainless steel screen, two large clamp holders (polymeric coated), base support, and a Spellman CZE1000R power supply (0 - 30,000 volts, Spellman High Voltage Electronic Corp., Hauppauge, NY). The physical set-up had the top clamp holder containing the glass pipet at approximately 12 inches from the base with the pipet tip pointing (pipet at approximately at 45 angle to base) toward the base. The wire was then placed in the top of the glass pipet and inserted until reaching the pipet tip where it remained during the procedure. The second clamp holder was placed at approximately 6 inches above the base for holding the screen (grounded target) approximately perpendicular to the axis of the glass pipet. The distance between the pipet tip and the grounded screen was approximately 10 cm. The positive lead from the high voltage power supply was attached to the wire hanging out the top end of the glass pipet while the negative lead (ground) was attached directly to the stainless steel screen. The glass pipet was then filled with the appropriate solution and the power supply turn on and adjusted until electrospinning was initiated (i.e. fibers shooting from the tip of the glass pipet). This stream (splay) of solution begins as a monofilament which between the pipet tip and the grounded target is converted to multifilaments (electric field driven phenomena). This allows for the production of a "web-like" structure to accumulate at the target site. Upon reaching the grounded target, the multifilaments collect and dry to form the 3-D interconnected polymeric matrix (fabric). The apparatus described is conceptually the same as the set-up illustrated in Figures 2A and 2B. All described studies and solutions are at room temperature. The fibers produced by these preliminary studies ranged from 1 - 100 microns in diameter with both polymeric solutions evaluated. The thickness of the matrices produced was not measured. Although, the thickness of the matrix that can be produced is dependent on the amount of polymer solution (spinning time) utilized and allowed to accumulate in a particular region. Thus, allowing the ability to produce a matrix with varying thickness across the sample. A scanning electron micrograph of the fiber forming the matrix is shown in Figure 1.

### Example 2 (Fabrication of a three dimensional segment of skeletal muscle)

An aligned collagen gel was prepared after the methods of Simpson et al., 1994 (Journal of Cell Physiology) on a silastic membrane (Speciality Manufacturing). Silastic membranes were sterilized in an autoclave and exposed to 2 minutes of electrical discharge to make the rubber more hydrophilic. Aligned collagen was then applied over the surface of the treated silastic rubber. In brief, 500 ul of 0.2 N HEPES was mixed with 500 ul of 10X MEM in a 50 ml centrifuge tube and placed on ice. Under sterile conditions 3.5 mls of Type I collagen (3 mg/ml in 0.012 HCL, Collagen Corporation) was layered over the top of the HEPES/10XMEM solution, mixed by inversion and diluted to a final volume of 10 mls with ice cold Phosphate Buffered Saline. A sterile and treated silastic membrane (70 mm X 30 mm) was placed in a 100 mm culture dish. One milliliter of ice cold collagen solution (final concentration 1.05 mg collagen/ml solution) was applied to the one end of the rectangular piece of silastic membrane. The collagen was pulled in a single continuous stroke across the long axis of the silastic membrane with a sterile cell scraper. The dish containing the silastic membrane was then tipped and the collagen was allowed to drain across the membrane along the axis that it was applied. The dish was covered and placed into a 37 degree Celsius incubator for 1 hour to allow the collagen to undergo polymerization. These procedures resulted in a thin layer of aligned collagen fibrils on the silastic membrane. The membranes were then allowed to dry in a moist atmosphere for 12-24 hours. This allows the collagen to partially dry down without pooling the collagen and disturbing the aligned collagen fibrils. The silastic membranes were then allowed to completely dry for an additional 30-60 minutes under a sterile laminar flow hood. Complete drying of the collagen anchors the fibrils to the rubber for further manipulation. Silastic membranes were used in these experiments solely to provide a support surface that could be easily manipulated for the fabrication of the engineered muscle.

A segment of silastic membrane (22 mm x 22 mm) containing uniformly arrayed collagen fibrils was cut and transferred to a sterile 35 mm culture dish. Cells of the mouse c2c12 skeletal muscle cell line were placed onto the silastic membranes and cultured for 3-5 days in DMEM-F12 (50:50 DMEM:F12 mix, supplemented with 10 % Horse serum, 5% FBS plus penstrep and gentimysin) and allowed to form a confluent culture of uniformly arrayed cells. These cultures served as a template layer for the further assembly of the cultures. A thin layer of silicon grease was placed on the underside of a piece of silastic rubber containing a culture of aligned c2c12 cells. The silicon grease serves as a non-toxic adhesive to anchor the culture to different locations within the culture chamber of a RCCS Bioreactor (Synthecon, Inc). In preliminary experiments several different locations in the bioreactor culture vessel were used in attempts to fabricate a three dimensional array of aligned muscle.

An aligned culture of c2c12 cells was placed on the back wall of the rotating bioreactor culture vessel, cell side up and held in place by a thin layer of silicon grease. The vessel was closed and then filled with culture media (50:50; DMEM:F12 mix, supplemented with 10 % Horse serum, 5% FBS plus penstrep and gentimysin). The syringe ports were opened and 1 million c2c12 cells were added as a single cells suspension to the bioreactor vessel. The device was mounted on the control axle that controls the rotational rate of the vessel. The entire device was then placed into a C02 incubator (37°C) and set to rotate at 3 revolutions per minute. At 24 hour intervals an additional 1 million c2c12 cells were added to the reactor chamber.

By setting the rate of rotation at a slow rate the cells added in suspension were gradually seeded out onto the template culture. After 48 hours of mixing in the vessel, the template cultures were isolated and prepared for electron microscopic examination. The cultures prepared in the RCCS reactor were composed of multiple layers of c2c12 cells arrayed along a common axis. Several different locations and conditions within the bioreactor were assayed for the ability to fabricate the multi-layered cultures. In this experimental run, it was found that cells arrayed on the back wall of the reactor in an orientation that was perpendicular to the direction of rotation was most effective. Other sites within the vessel also promoted the assembly of multilayered cultures, including the outer wall, central core and outer cylindrical wall. Aligned cultures that were oriented with the direction flow were also capable of promoting multilayer assembly, although not as effectively as the cultures oriented perpendicular to the direction of rotation.

In other experiments, an aligned collagen gel was prepared on a silastic membrane as described and placed directly into the RCCS bioreactor chamber (i.e. the experiments were designed to determine if the aligned collagen fibrils could promote multilayered assembly without first growing a confluent template layer of cells). Templates consisting of aligned collagen alone were about as equally effective as the templates containing confluent cell layers at promoting the assembly of the multilayered cultures. Again, these collagen templates were placed onto the bioreactor in different locations. They behaved identically as the confluent cell layers, i.e. collagen coated membranes of the back wall oriented perpendicular to the axis of rotation were most effective at promoting multilayer assembly.

A scanning electron micrograph of the muscle cells deposited on the aligned collagen is shown in Figure 4A.

### Example 3 (Electroaerosol Production of Extracellular Matrix)

An extracellular matrix in the form of a tube was made. Like the electrospinning described in Example 1, the electroaerosol process includes a polymer reservoir, spray nozzle and grounded mandrel. (See Figures 2A and 2B). In this experiment, the polymer reservoir and spray nozzle was a 1.0 ml syringe (minus plunger) and a simple plastic pipette tip (Gel Loading Tip, Fisher Scientific), respectively. The grounded mandrel was composed of stainless stell needle (18 gauge, length - 8 cm). Note: Prior to aerosol/matrix production, the mandrel was treated with a hexane solution saturated with Vaseline to allow easy removal of the formed construct from the mandrel. The polymeric solution used was polylactic/polyglycolyic acid (PLA/PGA; 50/50) at a concentration of 0.189 g/ml in methylene chloride. A fine wire was placed into the pipette tip as far as it would go. With this tip, the wire could not pass all the way through, thus approximately a quarter inch of the tip was cut-off at the point where the wire had passed through and became lodged. The wire in this experiment was charged to 12,000 volts (Spellman High Voltage Power Supply). Upon applying the electrical potential, the polymer aerosol began at the pipette tip and was directed towards the grounded mandrel. The aerosol was then collected around the mandrel. A total of 4 ml of the polymeric solution was used to create an extracellular matrix that could be used to construct, for instance, a nerve guide. Step one was to fill the reservoir/syringe with 1 ml of polymeric solution, charge the solution and allowing aerosol production. Upon emptying the reservoir, the mandrel was rotated 90 degrees (step 2) and step one was repeated. These steps were then repeated 4 time for the complete construct/nerve guide. Figures 8 to 11 are scanning electron micrographs of the extracellular matrix produced by this procedure.

The polymers evaluated -- polylactic/polyglycolyic acids (PLA/PGA; 50/50), give a soft, sponge yet fairly rigid construct with completed dried. As noted, the mechanical properties will depend on the polymer used, aerosol particle diameter, and overall mesh (matrix packing) structure.

Upon working with different material and mandrels, it should be noted that this technique could be able to form seamless constructs with bifurcations or other complex geometries (i.e. heart valves, vascular grafts, intestine, and cartilage (knee, nose, ear).

### Example 4 (Construction of a Vascular Prosthesis)

This example focuses on the development of a small diameter vascular prosthesis from collagen threads (Plain gut suture - collagen), arterial cellular components (smooth muscle cells (SMC), fibroblast cells (FB), and endothelial cells (EC)), collagen (Type I, III & IV), and fibronectin (All major components of arteries). Small diameter arteries such as the one described herein are composed of cylindrical layers (3-6 layers) of smooth muscle cells which are arranged in spirals of varying pitches. The arrangement of one example of a vascular prosthesis has the layers presented in Figure 12.

### -- Winding Apparatus

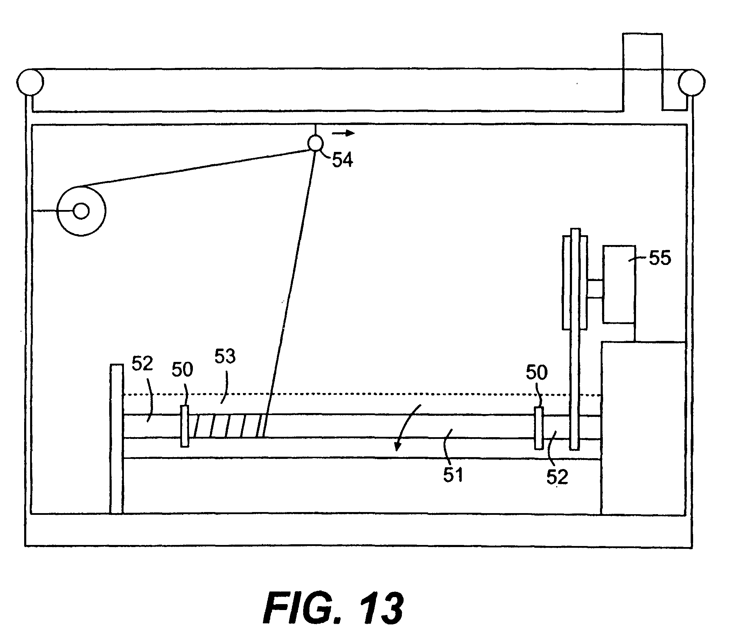

The winding apparatus (Figure 13) will be composed of two end supports 50, thin walled tubing 51, glass rod 52, bathing chamber 53, suture movement system 54, and a mandrel rotation system 55. The two end supports 50 will be bored centrally to match the external diameter of the thin walled tubing 51. The glass rod 52 will be placed through the thin walled tubing 51 during the collagen thread winding to maintain the graft cylindrical shape and be removed once in a bioreactor. The mandrel rotational system 55 will allow rotation of the graft mandrel at a specific speed. The suture movement and guidance system 54 will be mounted above the mandrel and will be composed of a rail and motor drive system to move the thread at a specific speed across the mandrel. The combination of the thread guidance speed and mandrel rotation speed will allow the control of the pitch of the collagen threads in layers 2-4 of the media. The pitch of layers 1 and 5 are high and will be prefabricated by weaving the suture between the mandrel end supports which will be designed to produce the desired pitch. The bathing chamber will allow the mandrel to be maintained in a saline or collagen solution during the winding procedure. The entire apparatus will be built from aluminum, 304 stainless steel (304SS), glass, and polycarbonate to allow sterilization by steam or ethylene oxide.

### -- Prosthetic Mandrel, Mandrel Holder, and Bioreactor

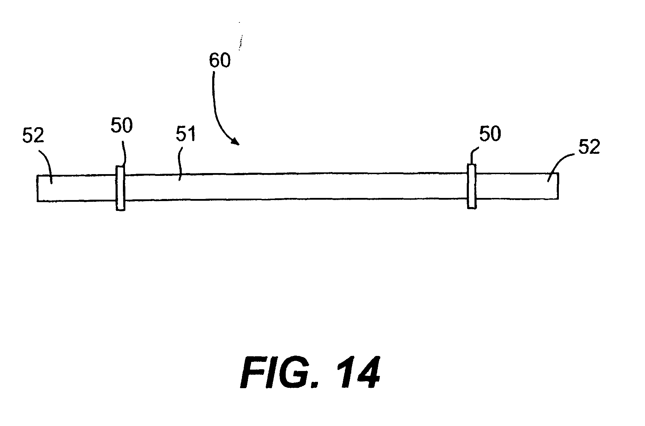

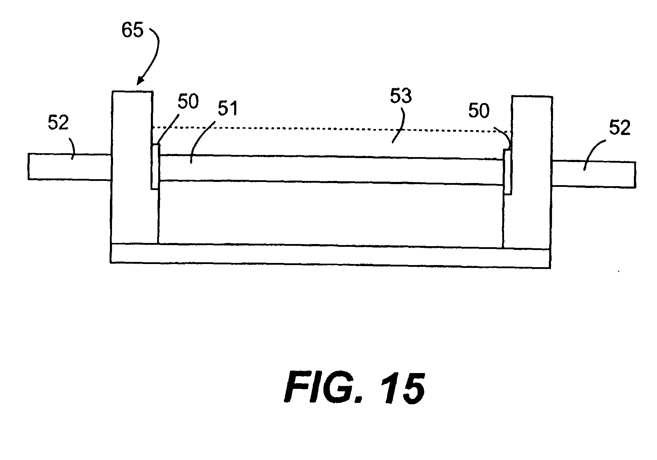

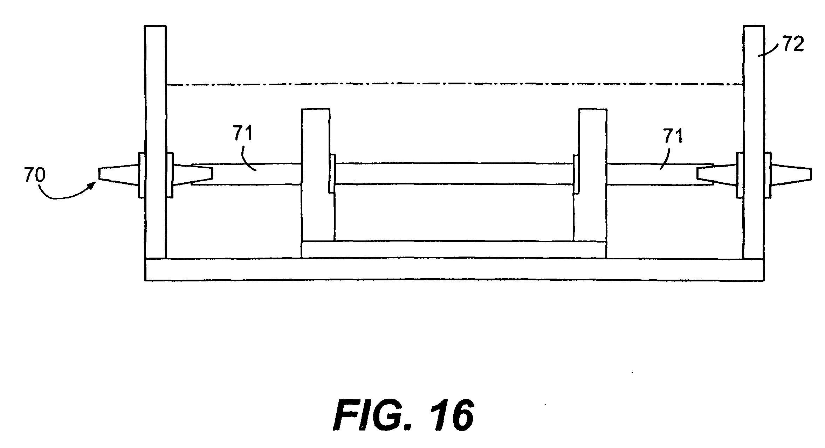

The mandrel 60 (Figure 14) will be composed of a glass rod 52, thin walled tubing 51, and two end supports 50. The end supports 50 will be composed of 304 SS. The exact composition (or whether it needs to be used at all) of the thin walled tubing 51 is not known at this time. The mandrel holder 65 (Figure 15) will maintain the vascular construct when placed within the bioreactor. It is primarily an end support system which doubles as a bathing chamber. The mandrel holder 65 will be filled with either saline or Medium-199. The mandrel holder 65 will be composed of polycarbonate which can be sterilized by either steam or ethylene oxide. The bioreactor (Figure 16) will be composed of tubing connectors 70, tubing 71, polycarbonate walls 72, and a pulsatile pump (not shown).

### --Methodology

The internal cylinder (lumen region) of the media will be developed by stringing (weaving) plain gut suture (0.23 mm diameter, L = 6 m; H. Hess & Co.) between the end supports at a high pitch (75°, almost parallel to the long axis of the vessel) with a 0.2 - 0.5 mm space between each suture. After weaving the plain gut suture, the mandrel will be placed into the bioreactor containing a suspension of human umbilical artery SMCs (UASMC). The suspension will be composed of 2x10⁶ UASMC/ml, collagen solution (46% type I & 54% type III; composition of normal vascular tissue; Imédex), 4X PBS, and distilled water to make a collagen concentration of 3 mg/ml. After 5 minutes at 37 °C, the gelification around the mandrel will have occurred and the mandrel transferred to its holder within the bioreactor. The well of the mandrel holder will contain a Medium-199 (M-199) (10% FBS) solution containing 2x10⁶ UASMC/ml. The collagen mixture will be allowed to form the initial cylindrical layer for 24 hours. During the 24 hour period in the bioreactor, the graft will have a pulsatile flow (flow rate: 90 ml/min; frequency 60 Hz; amplitude <5% of graft outer diameter) through the thin walled tubing of the mandrel for the first media layer. During subsequent media layers, the thin walled tubing will be removed to allow perfusion (nutrient delivery) to the media layers as well as direct mechanical forces. The advantage of this pulsatile flow during the media and adventitia development is that the cellular components will be exposed to circumferential (stretch-relaxation) forces. This is hoped to contribute to significant mechanical structural properties when compared to the vascular prostheses developed on mandrels under static conditions. After 24 hours, the second cylindrical layer will begin by winding the plain gut suture at a low pitch (15°; essentially perpendicular to the long axis of the vessel) with a spacing of approximately 0.2 - 0.5 mm. After the suture winding, the protocol is exactly as described for the initial layer. Layers 3-5 also follow the described protocol with suture pitches of -15°, 15°, and -75 °, respectively (Figure 12). All procedures will be performed under a laminar flow sterile workstation. The bioreactor apparatus (pulsatile flow system) will be housed in an incubator at 37°C, 5% CO₂ and constant humidity. No complications are expected due to the use of a bioabsorbable suture as the scaffolding. It has been shown that bioabsorbable suture (compared to non-absorbable) exhibits little to no anastomotic thrombus or hyperplasia along with less inflammation and scar tissue. It has been demonstrated that in vascular anastomoses, complete hydrolytic decomposition (@ 7 mo.) of the absorbable suture was followed by almost complete tissue regeneration of the vessel including the connective tissue of the media.

The adventitia equivalent structure will be developed by following the media development with fibroblast cells (FB)(5x10⁵ FB/ml) and collagen type I and III (3 mg/ml). The fibroblasts to be used will be human dermal fibroblasts (HDF). Three layers of the FB and collagen will be added to the media construct to build the adventitial layer. In small diameter arteries the adventitia can occupy up to half the thickness of the vascular wall. Thus, the necessity for multiple layers of the adventitial components.

The intima will be developed by endothelial cell (EC) seeding of the adventitia-media construct. Prior to EC seeding, the adventitia-media construct will be removed from the mandrel end supports and cannulated. The first step in EC seeding, neointimal development, is to coat the lumen surface with human fibronectin and collagen type IV. The EC seeding will be accomplished by injecting ECs (5x10⁶ EC/ml) suspended in M-199 (20% FBS) into the construct's lumen. The cannulas will then be sealed and the construct placed in a tube containing M-199 and 20% FBS overnight to allow adherence of the ECs to the construct. The tube and construct will be rotated at 1/8 r.p.m. during this time to allow an even distribution of the ECs on the luminal surface. After the EC adhesion period, the complete vascular prosthesis will be placed back in the bioreactor and exposed directly to physiologic pulsatile flow for 10 days (flow rate: 90 ml/min; frequency 60 Hz; amplitude <5% of graft outer diameter). The vascular construct will be bathed externally in M-199 and 10% FBS while the circulating media will be M-199 and 20% FBS. The circulating and bathing media will be replenished every two days.

Without further elaboration, it is believed that one skilled in the art can, using the proceeding description, utilize the present invention to its fullest extent. The preceding preferred specific embodiments are, therefore, to be construed as merely illustrative, and not limiting the remainder of the disclosure in any way whatsoever. The entire disclosure of all applications, patents, and publications, cited above and in the figures are hereby incorporated by reference in their entirety.

## Claims

1. An extracellular matrix comprising a matrix of electroaerosol droplets deposited on a substrate.

2. An extracellular matrix for supporting muscle comprising electrospun fibres.

3. An extracellular matrix as described in claim 1 or claim 2, wherein the electrospun fibres are cross-linked.

4. Electrospun collagen fibres.

5. A method of manufacturing an extracellular matrix comprising:
streaming an electrically-charged polymer solution on to a grounded target substrate under conditions effective to deposit polymer fibres which comprise collagen or polymer droplets on said substrate to form an extracellular matrix.

6. A method of claim 5, wherein the polymer droplets form a three-dimensional matrix.

7. A method of claim 5, wherein the polymer droplets comprise collagen.

8. A method of claim 5, comprising depositing a gel of aligned collagen fibers on said extracellular matrix.

9. A method according to claim 5 which is a method for forming a muscle fascial sheath comprising:
providing an electrically grounded substrate;
providing a reservoir of collagen solution wherein the reservoir has an orifice that allows the collagen solution to leave the reservoir;
electrically charging the collagen solution; and streaming the collagen onto the substrate to form a muscle fascial sheath.

10. A method of forming a vascular prosthesis comprising:
providing a mandrel, forming an extracellular matrix layer on the mandrel, and
depositing cells onto the extracellular matrix layer

11. The method of claim 10, wherein forming the extracellular matrix layer comprises winding a polymer fibre around the mandrel in a helical manner at a predetermined pitch.

12. The method described in claim 10, comprising forming a plurality of the extracellular matrix layers around the mandrel.

13. The method described in claim 11, wherein the predetermined pitch of the polymer fiber is different for each adjacent layer in the prosthesis.

14. The method described in any one of claims 10 to 13, wherein the extracellutar matrix comprises collagen gel.

15. An implant comprising the extracellular matrix as defined in any one of claims 1 to 3.

16. The implant of claim 15 which is a vascular prosthesis, a nerve guide or a fascial sheath.

17. The implant of claim 15 or claim 16, wherein the implant further comprises cells.

18. The implant of claim 17, wherein the cells are transformed cells, cultured cells, genetically engineered cells, non-human embryonic stem cells or non-embryonic stem cells.

19. The implant of any one of claims 15 to 18, wherein the implant further comprises DNA, growth factors, or chemotaxins.

20. The muscle implant of claim 15 comprising:
an extracellular matrix as defined in claim 2, and
a muscle cell layer, wherein the muscle cell layer is disposed on the extracellular matrix.

21. The muscle implant of claim 20 comprising a tendon comprising extruded fibres.

22. The muscle implant as described in claim 20 or claim 21 wherein the electrospun fibers comprise collagen fibers.

23. The muscle implant as described in any one of claims 20 to 22, wherein the electrospun fibers are cross-linked.

24. The muscle implant as described in any one of claims 20 to 22 comprising an oriented layer of collagen deposited onto the exttacellular matrix wherein the muscle cells are disposed onto the oriented layer of collagen.

25. A method of forming a muscle implant comprising:
providing a fascial sheath,
depositing a layer of collagen on the fascial sheath,
laminating a tendon onto the fascial sheath, and
depositing muscle cells onto the collagen.

26. The method described in claim 25, comprising:
aligning the layer of collagen on the fascial sheath.

27. The method described in claim 25 or claim 26 comprising:
laminating the tendon onto the fascial sheath in the same direction as the alignment of the collagen.

28. The method of any one of claims 25 to 27, wherein the fascial sheath is formed by electrospinning collagen fibers onto a substrate.

29. The method of any one of claims 25 to 28, wherein the tendon is formed by extruding collagen fibers.

30. The method of any one of claims 25 to 29, wherein the muscle cells are satellite muscle cells obtained from a patient into which the muscle implant will be placed.

31. A method of layering muscle cells on an extracellular matrix comprising:
providing an extracellular matrix,
placing the extracellular matrix inside a rotating wall bioreactor,
loading aculture medium into the bioreactor wherein the medium comprises muscle cells, and
running the bioreactor until muscle cells attach to the extracellular matrix.

32. The method described in claim 31, wherein the muscle cells attached to the extracellular matrix form multiple layers.

## Patentansprüche

1. Extrazelluläre Matrix, die eine Matrix aus auf einem Substrat abgelagerten Elektroaerosoltröpfchen umfasst.

2. Extrazelluläre Matrix zur Muskelunterstützung, die Elektrospinnfasern umfasst.

3. Extrazelluläre Matrix gemäß der Beschreibung in Anspruch 1 oder 2, wobei die Elektroaerosoltröpfchen oder Elektrospinnfasern vernetzt sind.

4. Kollagen-Elektrospinnfasern.

5. Verfahren zur Herstellung einer extrazellulären Matrix, das das Aufstrahlen bzw. -strömen einer elektrisch geladenen Polymerlösung auf ein geerdetes Zielsubstrat unter Bedingungen, die zur Ablagerung von Kollagen umfassenden Polymerfasern oder von Polymertröpfchen auf dem Substrat unter Bildung einer extrazellulären Matrix wirksam sind, umfasst.

6. Verfahren nach Anspruch 5, wobei die Polymertröpfchen eine dreidimensionale Matrix bilden.

7. Verfahren nach Anspruch 5, wobei die Polymertröpfchen Kollagen umfassen.

8. Verfahren nach Anspruch 5, das die Ablagerung eines Gels von ausgerichteten Kollagenfasern auf der extrazellulären Matrix umfasst.

9. Verfahren nach Anspruch 5, das ein Verfahren zur Bildung einer Muskelfaszienhülle ist, das umfasst:
das Bereitstellen eines elektrisch geerdeten Substrats;
das Bereitstellen eines Reservoirs einer Kollagenlösung, wobei das Reservoir eine Öffnung besitzt, die es ermöglicht, dass die Kollagenlösung das Reservoir verlässt;
das elektrische Aufladen der Kollagenlösung; und
das Aufstrahlen bzw. -strömen des Kollagens auf das Substrat zur Bildung einer Muskelfaszienhülle.

10. Verfahren zur Bildung einer Gefäßprothese, das umfasst:
das Bereitstellen eines Formkerns,
das Bilden einer extrazellulären Matrixschicht auf dem Formkern und
die Ablagerung von Zellen auf der extrazellulären Matrixschicht.

11. Verfahren nach Anspruch 10, wobei das Bilden der extrazellulären Matrixschicht das Wickeln einer Polymerfaser um den Formkern in schraubenförmiger Weise mit einer vorgegebenen Schraubensteigung umfasst.

12. Verfahren gemäß der Beschreibung in Anspruch 10, das das Bilden einer Mehrzahl der extrazellulären Matrixschichten um den Formkern umfasst.

13. Verfahren gemäß der Beschreibung in Anspruch 11, wobei die vorgegebene Schraubensteigung der Polymerfaser für jede angrenzende Schicht in der Prothese verschieden ist.

14. Verfahren gemäß der Beschreibung in einem der Ansprüche 10 bis 13, wobei die extrazelluläre Matrix ein Kollagengel umfasst.

15. Implantat, das die extrazelluläre Matrix gemäß der Definition in einem der Ansprüche 1 bis 3 umfasst.

16. Implantat nach Anspruch 15, das eine Gefäßprothese, eine Nervenführung oder eine Faszienhülle ist.

17. Implantat nach Anspruch 15 oder Anspruch 16, wobei das Implantat ferner Zellen umfasst.

18. Implantat nach Anspruch 17, wobei die Zellen transformierte Zellen, gezüchtete Zellen, gentechnisch veränderte Zellen, nichthumane embryonale Stammzellen oder nichtembryonale Stammzellen sind.

19. Implantat nach einem der Ansprüche 15 bis 18, wobei das Implantat ferner DNA, Wachstumsfaktoren oder Chemotaxine umfasst.

20. Muskelimplantat nach Anspruch 15, das eine extrazelluläre Matrix gemäß der Definition in Anspruch 2 und eine Muskelzellschicht umfasst, wobei sich die Muskelzellschicht auf der extrazellulären Matrix befindet.

21. Muskelimplantat nach Anspruch 20, das eine extrudierte Fasern umfassende Sehne umfasst.

22. Muskelimplantat gemäß der Beschreibung in Anspruch 20 oder Anspruch 21, wobei die Elektrospinnfasern Kollagenfasern umfassen.

23. Muskelimplantat gemäß der Beschreibung in einem der Ansprüche 20 bis 22, wobei die Elektrospinnfasern vernetzt sind.

24. Muskelimplantat gemäß der Beschreibung in einem der Ansprüche 20 bis 22, das eine auf der extrazellulären Matrix abgelagerte orientierte Kollagenschicht umfasst, wobei die Muskelzellen auf der orientierten Kollagenschicht angeordnet sind.

25. Verfahren zur Bildung eines Muskelimplantats, das umfasst:
das Bereitstellen einer Faszienhülle,
die Ablagerung einer Kollagenschicht auf der Faszienhülle,
das Laminieren einer Sehne an die Faszienhülle und
die Ablagerung von Muskelzellen auf das Kollagen.

26. Verfahren gemäß der Beschreibung in Anspruch 25, das das Ausrichten der Kollagenschicht auf der Faszienhülle umfasst.

27. Verfahren gemäß der Beschreibung in Anspruch 25 oder Anspruch 26, das das Laminieren der Sehne an die Faszienhülle in der gleichen Richtung wie die Ausrichtung des Kollagens umfasst.

28. Verfahren nach einem der Ansprüche 25 bis 27, wobei die Faszienhülle durch Elektrospinnen von Kollagenfasern auf ein Substrat gebildet wird.

29. Verfahren nach einem der Ansprüche 25 bis 28, wobei die Sehne durch Extrudieren von Kollagenfasern gebildet wird.

30. Verfahren nach einem der Ansprüche 25 bis 29, wobei die Muskelzellen Satellitenmuskelzellen sind, die von einem Patienten erhalten wurden, dem das Muskelimplantat eingepflanzt wird.

31. Verfahren zur Schichtbildung von Muskelzellen auf einer extrazellulären Matrix, das umfasst:
das Bereitstellen einer extrazellulären Matrix,
das Platzieren der extrazellulären Matrix im Inneren eines Rotationswandbioreaktors,
das Beladen des Bioreaktors mit einem Kulturmedium, wobei das Medium Muskelzellen umfasst, und
das Betreiben des Bioreaktors, bis Muskelzellen an der extrazellulären Matrix haften.

32. Verfahren gemäß der Beschreibung in Anspruch 31, wobei die an der extrazellulären Matrix angehefteten Muskelzellen mehrere Schichten bilden.

## Revendications

1. Matrice extracellulaire comprenant une matrice de gouttelettes d'électroaérosol déposées sur un substrat.

2. Matrice extracellulaire destinée à soutenir du muscle comprenant des fibres électrofilées.

3. Matrice extracellulaire telle que décrite dans la revendication 1 ou la revendication 2, dans laquelle les gouttelettes d'électroaérosol ou les fibres électrofilées sont réticulées.

4. Fibres collagènes électrofilées.

5. Procédé de fabrication d'une matrice extracellulaire comprenant :
l'envoi d'un jet d'une solution polymère chargée
électriquement sur un substrat cible mis à la terre dans des conditions efficaces pour déposer des fibres de polymère qui comprennent du collagène ou des gouttelettes de polymère sur ledit substrat pour former une matrice extracellulaire.

6. Procédé selon la revendication 5, dans lequel les gouttelettes de polymère forment une matrice tridimensionnelle.

7. Procédé selon la revendication 5, dans lequel les gouttelettes de polymère comprennent du collagène.

8. Procédé selon la revendication 5, comprenant le dépôt d'un gel de fibres collagènes alignées sur ladite matrice extracellulaire.

9. Procédé selon la revendication 5 qui est un procédé pour former une gaine fasciale musculaire comprenant :
la fourniture d'un substrat électriquement mis à la terre ;
la fourniture d'un réservoir de solution de collagène dans lequel le réservoir a un orifice qui permet à la solution de collagène de s'évacuer du réservoir ;
la charge électrique de la solution de collagène ; et
l'envoi d'un jet de collagène sur le substrat pour former une gaine fasciale musculaire.

10. Procédé de formation d'une prothèse vasculaire comprenant :
la fourniture d'un mandrin,
La formation d'une couche de matrice extracellulaire sur le mandrin, et
le dépôt de cellules sur la couche de matrice extracellulaire.

11. Procédé selon la revendication 10, dans lequel la formation de la couche de matrice extracellulaire comprend l'enroulement d'une fibre de polymère autour du mandrin d'une manière hélicoïdale à un pas prédéterminé.

12. Procédé décrit dans la revendication 10, comprenant la formation d'une pluralité de couches de matrice extracellulaire autour du mandrin.

13. Procédé décrit dans la revendication 11, dans lequel le pas prédéterminé de la fibre polymère est différent pour chaque couche adjacente dans la prothèse.

14. Procédé décrit dans l'une quelconque des revendications 10 à 13, dans lequel la matrice extracellulaire comprend du gel de collagène.

15. Implant comprenant la matrice extracellulaire telle que définie dans l'une quelconque des revendications 1 à 3.

16. Implant selon la revendication 15, qui est une prothèse vasculaire, un guide de nerf ou une gaine fasciale.

17. Implant selon la revendication 15 ou la revendication 16, dans lequel l'implant comprend en outre des cellules.

18. Implant selon la revendication 17, dans lequel les cellules sont des cellules transformées, des cellules cultivées, des cellules élaborées génétiquement, des cellules souches embryonnaires non humaines ou des cellules souches non embryonnaires.

19. Implant selon l'une quelconque des revendications 15 à 18, dans lequel l'implant comprend en outre de l'ADN, des facteurs de croissance ou des chimiotaxines.

20. Implant musculaire selon la revendication 15, comprenant :
une matrice extracellulaire telle que définie dans la revendication 2, et
une couche de cellules musculaires, dans laquelle la couche de cellules musculaires est disposée sur la matrice extracellulaire.

21. Implant musculaire selon la revendication 20 comprenant un tendon comprenant des fibres extrudées.

22. Implant musculaire tel que décrit dans la revendication 20 ou la revendication 21, dans lequel les fibres électrofilées comprennent des fibres de collagène.

23. Implant musculaire tel que décrit dans l'une quelconque des revendications 20 à 22, dans lequel les fibres électrofilées sont réticulées.

24. Implant musculaire tel que décrit dans l'une quelconque des revendications 20 à 22, comprenant une couche orientée de collagène déposée sur la matrice extracellulaire, dans lequel les cellules musculaires sont disposées sur la couche orientée de collagène.

25. Procédé de formation d'un implant musculaire comprenant :
la fourniture d'une gaine fasciale,
le dépôt d'une couche de collagène sur la gaine fasciale,
la formation d'un stratifié avec un tendon que l'on applique sur la gaine fasciale, et
le dépôt de cellules musculaires sur le collagène.

26. Procédé décrit dans la revendication 25, comprenant :
l'alignement de la couche de collagène sur la gaine fasciale.

27. Procédé décrit dans la revendication 25 ou la revendication 26, comprenant :
la formation d'un stratifié avec un tendon que l'on applique sur la gaine fasciale dans la même direction que celle de l'alignement du collagène.

28. Procédé selon l'une quelconque des revendications 25 à 27, dans lequel la gaine fasciale est formée par électrofilage de fibres de collagène sur un substrat.

29. Procédé selon l'une quelconque des revendications 25 à 28, dans lequel le tendon est formé par extrusion de fibres de collagène.

30. Procédé selon l'une quelconque des revendications 25 à 29, dans lequel les cellules musculaires sont des cellules musculaires satellites obtenues chez un patient dans lequel sera placé l'implant musculaire.

31. Procédé de répartition en couches des cellules musculaires sur une matrice extracellulaire comprenant :
la fourniture d'une matrice extracellulaire,
la mise en place de la matrice extracellulaire à l'intérieur d'un bioréacteur à paroi rotative,
le chargement d'un milieu de culture dans le bioréacteur dans lequel le milieu comprend des cellules musculaires, et
le fonctionnement du bioréacteur jusqu'à ce que des cellules musculaires se fixent à la matrice extracellulaire.

32. Procédé décrit dans la revendication 31, dans lequel les cellules musculaires fixées à la matrice extracellulaire forment des couches multiples.
